# EUROPÄISCHE PATENTSCHRIFT

(11) **EP 1 932 495 B1**
(45) Veröffentlichungstag und Bekanntmachung des Hinweises auf die Patenterteilung: **17.02.2010**
(21) Anmeldenummer: 07122947.0
(22) Anmeldetag: 11.12.2007
(51) Int. Cl.: A61F 2/46, A61F 2/30

(54) **Bestimmung einer Gelenkorientierung für eine Implantation**
Joint alignment test for an implant
Détermination de l'orientation d'une articulation pour une implantation

(30) Priorität: 12.12.2006 EP 06025672
(43) Veröffentlichungstag der Anmeldung: 18.06.2008
(73) Patentinhaber: BrainLAB AG, 85622 Feldkirchen (DE)
(72) Erfinder: Dick, Robert, 80686, München (DE); Dohmen, Lars, 81549, München (DE)
(74) Vertreter: Schwabe - Sandmair - Marx

(56) Entgegenhaltungen:
- EP-A1- 1 402 855
- WO-A-02/080824
- DE-A1- 19 709 960
- US-B1- 6 205 411

## Beschreibung

Die vorliegende Erfindung betrifft die Bestimmung einer Orientierung eines anatomischen Gelenks und/oder eine zur Implantation eines künstlichen Gelenks geeignete Orientierung des künstlichen Gelenks, insbesondere die Planung der Implantation eines künstlichen Gelenks sowie eine Vorrichtung zum Bestimmen der Orientierung zur Implantation eines künstlichen Gelenks. Das künstliche Gelenk wird implantiert, um ein bereits im menschlichen oder tierischen Körper vorhandenes Gelenk zu ersetzen. Dieses zu ersetzende Gelenk wird im Folgenden anatomisches Gelenk genannt. Das anatomische Gelenk kann aus bestimmten Gründen, z.B. Unfall oder Krankheit (z.B. Osteoporose) defekt oder nur noch eingeschränkt funktionsfähig sein oder Schmerzen verursachen. Um Komplikationen nach der Implantation eines künstlichen Gelenks möglichst zu vermeiden, ist eine Implantation des künstlichen Gelenks mit einer geeigneten Orientierung von Vorteil.

Das Dokument US-A-6,205,411 stellt der nächste Stand der Technik dar.

Aufgabe der Erfindung ist es, ein Verfahren und eine Vorrichtung bereitzustellen, um die Planung einer Implantation eines künstlichen Gelenks mit einer geeigneten Orientierung und/oder an einer geeigneten Lage zu unterstützen.

Vorstehende Aufgabe wird durch die Gegenstände der unabhängigen Ansprüche gelöst. Vorteilhafte Weiterbildungen gehen aus den Unteransprüchen hervor.

Das künstliche Gelenk, das erfindungsgemäß ein anatomisches Gelenk, wie z.B. ein Hüftgelenk oder ein Knie-, Fuß-, Finger- oder Schultergelenk ersetzen soll, hat vorzugsweise mindestens zwei Teile, die relativ zueinander beweglich sind und eine Gelenkverbindung eingehen können. Ein Beispiel für einen (ersten) Teil eines künstlichen Gelenks ist eine Gelenkpfanne und ein Beispiel für den dazu gehörigen anderen (zweiten) Teil ist ein Gelenkkopf, der beispielsweise an einem Schaft angebracht ist. Kopf und Pfanne sind zueinander passend ausgebildet. Beispielsweise kann der erste Teil die Gelenkpfanne und der zweite Teil der Gelenkkopf sein. Die Benennung "erste" und "zweite" ist hierin willkürlich und kann auch anders herum gewählt werden. Sie bringt lediglich zum Ausdruck, dass es sich um zwei kooperierende Teile eines Gelenks handelt.

Der erste Teil des künstlichen Gelenks ist vorzugsweise ausgebildet, um in eine erste Körperstruktur (z.B. ein Knochen oder ein Knorpel oder eine Mischung daraus) implantiert zu werden. Beispielsweise kann eine künstliche Gelenkpfanne in einen Beckenknochen implantiert werden, wobei die künstliche Gelenkpfanne beispielsweise den ersten Teil darstellt und das Becken (Pelvis) die erste Körperstruktur. Weiter kann bei einer Totalendoprothese der künstliche Kugelgelenkkopf zusammen mit einem Schaft in einem Oberschenkelknochen (Femur) implantiert werden, wobei beispielsweise der Kugelgelenkkopf zusammen mit dem Schaft den zweiten Teil des künstlichen Gelenks darstellt und der Oberschenkelknochen die zweite Körperstruktur darstellt.

Erfindungsgemäß wird vorzugsweise aber nicht obligatorisch ein Implantat-Datensatz bereitgestellt. Dieser kann beispielsweise in einem Speicher abgespeichert sein oder über eine Eingabeschnittstelle (z.B. Netzwerkanschluss, Tastatur etc.) eingegeben werden. Der Implantatdatensatz umfasst vorzugsweise Daten, die eine Bewegbarkeit des ersten Teils relativ zum zweiten Teil betreffen.

Was der Begriff "Bewegbarkeit" umfasst, wird im Folgenden anhand des künstlichen Gelenks erläutert, trifft aber analog auch auf das anatomische Gelenk zu, wobei der erste Teil des künstlichen Gelenks der ersten Körperstruktur entspricht und der zweite Teil des künstlichen Gelenks der zweiten Körperstruktur. Der Begriff umfasst insbesondere einen Bewegungsbereich des künstlichen Gelenks, also beispielsweise die möglichen Winkel, die der erste Teil des künstlichen Gelenks relativ zum zweiten Teil einnehmen kann, insbesondere maximal mögliche Winkel. Diese Winkel können für Ebenen definiert sein, die beispielsweise senkrecht zueinander stehen und insbesondere charakteristische Ebenen (Schlüsselebenen) des künstlichen Gelenks darstellen. Insbesondere können sie maximale Bewegungsbereiche darstellen, die das künstliche Gelenk erlaubt. Zum Beispiel kann es im Rahmen einer Relativbewegung zu einem Anstoßen des zweiten Teils (z.B. Kugeln mit Schaft) an dem ersten Teil (z.B. Pfanne) kommen. Die Bewegungsbereiche können z.B. durch Winkel beschrieben werden, die für bestimmte Ebenen definiert werden, in denen insbesondere eine Bewegung des ersten Teils relativ zum zweiten Teil möglich ist. Weiter kann die Bewegbarkeit mögliche Lagen, insbesondere Extremlagen oder Schlüssellagen umfassen, bei denen der erste Teil relativ zum zweiten Teil eine bestimmte Lage, insbesondere Extremlage oder Schlüssellage einnimmt. Ein Beispiel für eine Extremlage ist gegeben, wenn der erste Teil relativ zum zweiten Teil den Rand eines möglichen Bewegungsbereichs erreicht hat und die beiden Teile aneinander anstoßen, so dass eine Bewegung nur noch in eine Richtung, weg von der Extremlage (Anstoßpunkt) und zurück zu einer bereits vorher eingenommenen Relativlage im Rahmen einer üblichen Gelenkbewegung möglich ist. Eine übliche Gelenkbewegung ist eine Bewegung, bei der die beiden Teile des Gelenks (erster und zweiter Teil) nicht getrennt werden sondern Bewegungen ausführen, wie sie insbesondere den (gesunden) Bewegungen des anatomischen Gelenks entsprechen. Die Lagen können z.B. in einem Bezugssystem beschrieben werden, das im ersten oder im zweiten Teil verankert ist und beispielsweise mit kartesischen Koordinaten oder Kugelkoordinaten oder Winkeln relativ zu den Ebenen beschrieben werden. Insbesondere können die relativen Lagen von Symmetrieachsen oder -ebenen im ersten Teil relativ zu Symmetrieachsen oder -ebenen im zweiten Teil beschrieben werden oder die Positionen bestimmter Elemente (z.B. Landmarken) des ersten Teils relativ zu Positionen bestimmter Elemente des zweiten Teils (beispielsweise der tiefste Punkt einer schalenförmigen Vertiefung eines Pfannengelenks) beschrieben werden.

Weiter kann der Implantat-Datensatz Daten über die Geometrie des anatomischen Gelenks umfassen. Daten über die Geometrie sind beispielsweise Daten über die Form und/oder Gestaltung des künstlichen Gelenks. Die Bewegbarkeit kann durch die Geometrie und/oder durch das Material oder die Materialien beeinflusst werden, die das künstliche Gelenk umfasst oder aus denen es gebildet ist, insbesondere die Elastizität, Flexibilität und/oder Starrheit der Materialien. Letztere Eigenschaften und/oder die Geometrie können insbesondere beeinflussen, mit welchen Kräften sich die Teile des künstlichen Gelenks (erster und zweiter Teil) voneinander trennen lassen. Die Bewegbarkeit umfasst zudem vorzugsweise Informationen über eine mögliche relative Bewegung des ersten Teils und zweiten Teils im Normalfall, bei welchem das künstliche Gelenk eine Bewegung des gesunden anatomischen Gelenks nachahmt. Vorzugsweise umfasst die Bewegbarkeit auch Information über eine Bewegung des ersten Teils relativ zu dem zweiten Teil, bei dem sich das erste Teil vom zweiten Teil trennt, wie dies insbesondere unerwünscht ist und wie dies bei einer Luxation der Fall sein kann. Diese unerwünschte relative Bewegung wird insbesondere auch durch die Geometrie des künstlichen Gelenks beeinflusst. Insbesondere beeinflusst die Geometrie den Grad des Widerstandes, den das künstliche Gelenk gegen eine unerwünschte Trennung des ersten und zweiten Teils entgegensetzt.

Der Implantat-Datensatz umfasst somit vorzugsweise auch Daten über die Hemmung einer Bewegung, die eine Trennung des ersten Teils und des zweiten Teils bewirkt, also insbesondere die Hemmung einer Luxationsbewegung. Insbesondere umfasst er Daten über den Grad der Hemmung einer derartigen Bewegung oder über den Widerstand, der einer solchen Bewegung entgegengesetzt wird. Der Grad der Hemmung und/oder der Widerstand, kann sich insbesondere aus der Geometrie des künstlichen Gelenks ergeben und kann insbesondere durch geometrische Eigenschaften ausgedrückt werden, wie weiter unten bei der detaillierten Beschreibung näher ausgeführt wird. Auch kann er durch Kräfte, beispielsweise Reibungskräfte oder Haltekräfte ausgedrückt werden.

Der Implantat-Datensatz kann beispielsweise einer Datenbank entnommen werden, die für die einzelnen Typen von Implantaten die entsprechenden Daten speichert. Auch können die Eigenschaften des Implantats vermessen werden, indem beispielsweise Relativbewegungen zwischen dem ersten und zweiten Teil ausgeführt werden und indem zum Beispiel bei einer Stellung (z.B. Schlüsselstellung) oder bei bestimmten relativen Stellungen des ersten und zweiten Teils versucht wird, den ersten Teil von dem zweiten Teil zu trennen und die entsprechenden Kräfte, die hierzu notwendig sind, gemessen werden. Diese Kräfte können dann als Daten dem Implantat-Datensatz hinzugefügt werden bzw. diesen bilden.

Erfindungsgemäß werden vorzugsweise Körperstruktur-Daten bereitgestellt, die insbesondere patientenspezifisch sind. Diese umfassen insbesondere Informationen über eine Bewegbarkeit des anatomischen Gelenks, also insbesondere der ersten und/oder zweiten Körperstruktur. Der Begriff der "Bewegbarkeit" wird hierbei analog zu dem oben erläuterten Begriff der Bewegbarkeit verstanden und umfasst insbesondere Informationen über mögliche relative Bewegungen und/oder relative Lagen der ersten Körperstruktur relativ zur zweiten Körperstruktur.

Vorzugsweise umfassen die Körperstruktur-Daten Informationen über die Lage der ersten Körperstruktur und/oder zweiten Körperstruktur, insbesondere über eine relative Orientierung der ersten Körperstruktur relativ zu der zweiten Körperstruktur. Die Lage und/oder Orientierung kann in einem vorbestimmten Bezugssystem beschrieben werden, z.B. in einem Bezugssystem des Patienten, in einem Bezugssystem eines verwendeten Navigationssystems (z.B. Bezugssystem, in dem eine Detcktionseinrichtung, z.B. Kamera des Navigationssystems ruht, in einem Bezugssystem, in dem der Operationsraum ruht usw. beschrieben werden) oder in einem Bezugssystem, das in einer der Körperstrukturen ruht. Umfassen beispielsweise die Informationen über die Lage sowohl Informationen über die Lage der ersten als auch über die Lage der zweiten Körperstruktur, so kann die Lage zumindest einer der beiden Körperstrukturen in einem Bezugssystem beschrieben werden, in dem die andere Körperstruktur ruht.

Die Körperstruktur-Daten, die die Bewegbarkeit der ersten relativ zur zweiten Körperstruktur betreffen, umfassen insbesondere mindestens einen Bewegungsbereich des anatomischen Gelenks, also insbesondere relative Bewegungen der ersten Körperstruktur relativ zur zweiten Körperstruktur. Insbesondere umfassen sie mindestens einen maximalen möglichen Bewegungsbereich, insbesondere zwei Bewegungsbereiche. Die Bewegungsbereiche werden bevorzugt für bestimmte Bewegungsrichtungen bzw. Bewegungsebenen beschrieben. Im Falle eines Hüftgelenkimplantats, erfolgt die Beschreibung beispielsweise in Richtung der Extension/Flexion des anatomischen Gelenks und/oder in der Richtung der Adduktion und Abduktion des Gelenks. Insbesondere umfasst die Bewegbarkeit eine oder mehrere bestimmte Schlüssellagen der ersten Körperstruktur relativ zur zweiten Körperstruktur, also beispielsweise eine Angabe über eine Extremlage und/oder über einen maximalen Abduktionswinkel zwischen der ersten Körperstruktur (Gelenkpfanne) und der zweiten Körperstruktur (Gelenkkopf), um für diesen Fall das Risiko einer möglichen Luxation zu berechnen oder abzuschätzen, wie weiter unten aufgeführt wird.

Die Körperstruktur-Daten umfassen insbesondere Daten über Winkel und/oder Winkelverhältnisse. Die Winkel ergeben sich durch die Bewegung der ersten Körperstruktur relativ zur zweiten Körperstruktur. Die Winkelverhältnisse ergeben sich insbesondere durch Bezugnahme auf eine neutrale Relativlage der ersten Körperstruktur zur zweiten Körperstruktur. Die Körperstruktur-Daten hängen insbesondere von der Orientierung des anatomischen Gelenks ab. Insbesondere erlauben sie die Berechnung der Orientierung des anatomischen Gelenks, falls in die Berechnung zusätzlich einfließt, welcher Winkel zwischen den Bewegungsendpunkten liegt. Insbesondere umfassen die Körperstruktur-Daten Daten, die eine Funktion der Orientierung des anatomischen Gelenks sind. Die weiter unten beschriebenen Winkelverhältnisse stellen ein Beispiel für derartige Daten dar, da sich je nach Orientierung des anatomischen Gelenks die Winkelverhältnisse ändern würden. Weitere Beispiele stellen die ebenfalls weiter unten beschriebenen absoluten Winkelwerte dar. Die Körperstruktur-Daten können somit sowohl Daten umfassen, die eine Funktion der Orientierung des anatomischen Gelenks darstellen. Sie können aber auch Daten umfassen, die die Orientierung des anatomischen Gelenks direkt darstellen. Ein Beispiel hierfür ist der weiter unten beschriebene Mittelpunkt des Bewegungsbereichs.

Die Informationen über die Bewegbarkeit können insbesondere auch Daten über die Geometrie des anatomischen Gelenks umfassen, insbesondere Daten über die Gestalt und/oder Form des anatomischen Gelenks. Diese Daten können durch Diagnoseverfahren, wie z.B. Kernspinaufnahmen, Röntgenaufnahmen, Computertomographieaufnahmen (CT) usw. gewonnen werden. Insbesondere können Informationen über die Bewegbarkeit Daten über die Hemmung einer Bewegung und/oder den Widerstand gegen eine Bewegung der ersten und/oder zweiten Körperstruktur umfassen. Insbesondere können von dem Betriff der Bewegbarkeit Daten über Bänderspannungen, Lage von Bändern und Gewebestrukturen, die die Bewegung und den Bewegungsbereich des anatomischen Gelenks beeinflussen, umfasst sein. Insbesondere können typische (übliche) Werte in den Daten über die Bewegbarkeit enthalten sein, z.B. typische Werte über den Widerstand gegen eine Luxation und/oder typische Werte über Bewegungsbereiche, wie sie z.B. typischerweise (üblicherweise) bei einem Menschen gegeben sind.

Vorzugsweise wird erfindungsgemäß basierend auf den Körperstruktur-Daten eine Orientierung des anatomischen Gelenks bestimmt, die für die Planung der Implantation des künstlichen Gelenks verwendbar ist, und insbesondere die Bestimmung einer für die Implantation geeignete Orientierung des künstlichen Gelenks erlaubt. Erfindungsgemäß kann also eine Bestimmung der Orientierung des anatomischen Gelenks erfolgen. Diese bestimmte Orientierung kann dann von einem Planungsverfahren oder einem Arzt verwendet werden, um eine Implantation zu planen, insbesondere eine geeignete Lage und/oder Orientierung des künstlichen Gelenks zu bestimmen. Auch kann erfindungsgemäß eine geeignete Orientierung des künstlichen Gelenks direkt aus den Körperstrukturdaten bestimmt werden, indem beispielsweise Bewegungsbereiche des anatomischen und künstlichen Gelenks abgeglichen werden, ohne dass die Orientierung des anatomischen Gelenks bestimmt wird. Die Bestimmung der Orientierung des anatomischen Gelenks kann aber auch ein Zwischenschritt zur Bestimmung der geeigneten Orientierung des künstlichen Gelenks sein.

Die bestimmte Orientierung des anatomischen Gelenks kann dann als Anhaltspunkt für eine zur Implantation geeigneten Orientierung des künstlichen Gelenks verwendet werden. Insbesondere kann die Implantation so geplant werden, dass die Orientierung des künstlichen Gelenks mit der bestimmten Orientierung des anatomischen Gelenks übereinstimmt. Eine Übereinstimmung ist jedoch nicht obligatorisch, insbesondere wenn aus medizinischen Gründen für das künstliche Gelenk eine vom anatomischen Gelenk abweichende Orientierung gewünscht wird.

Vorzugsweise erfolgt die Bestimmung der geeigneten Orientierung des künstlichen Gelenks auch basierend auf dem mindestens einen Implantat-Datensatz. Dabei ist die bestimmte Orientierung des künstlichen Gelenks geeignet für eine Implantation des ersten und/oder zweiten Teils des künstlichen Gelenks in einer Körperstruktur. Insbesondere ist diese Orientierung geeignet für eine Implantation des ersten Teils des künstlichen Gelenks (z.B. künstliche Gelenkpfanne) in eine erste Körperstruktur (z.B. Beckenknochen) und/oder für eine Implantation eines zweiten Teils des künstlichen Gelenks (z.B. Kugel mit Schaft) in eine zweite Körperstruktur (z.B. Oberschenkelknochen).

Die geeignete Orientierung kann z.B. als Gerade oder als Vektor in verschiedenen Bezugssystemen beschrieben werden, wie sie schon oben in Zusammenhang mit der Lage der ersten und zweiten Körperstruktur diskutiert wurden. Insbesondere kann die geeignete Orientierung in einem Bezugssystem beschrieben werden, in dem der Patient ruht, oder in dem der Operationsraum ruht, oder in dem ein Navigationssystem bzw. dessen Detektionssystem ruht. Insbesondere kann die geeignete Orientierung des künstlichen Gelenks auch in einem Bezugssystem beschrieben werden, in dem die erste Körperstruktur oder die zweite Körperstruktur ruht oder in dem einer der Teile des künstlichen Gelenks ruht. Insbesondere kann die geeignete Orientierung unter Bezugnahme auf Symmetrieebenen oder Landmarken der ersten und/oder zweiten Körperstruktur angegeben werden, wie beispielsweise die Symmetrieebenen des Beckenknochens.

Die Orientierung eines Gelenks wird vorzugsweise über dessen Bewegbarkeit, insbesondere Bewegungsbereich und/oder dessen Symmetrieeigenschaften (z.B. Symmetrieachsen, Symmetriepunkte, Symmetrieebenen, insbesondere Längsachsen eines Gelenkteils) bestimmt.

Beispielsweise kann die Orientierung durch einen Vektor beschrieben werden, der seinen Ursprung in der Mitte des Grundes der schalenförmigen Vertiefung des Acetabulums hat. Alternativ kann er beispielsweise seinen Ursprung auch dort haben, wo sich der Drehpunkt des anatomischen Gelenks befindet.

Die Orientierung eines Gelenks, insbesondere des anatomischen Gelenks und/oder des künstlichen Gelenks kann beispielsweise wie folgt bestimmt werden. Ein Teil eines zweiteiligen Gelenks wird festgehalten, während der andere Teil den Rand des zulässigen Bewegungsbereichs des Gelenks abfährt. Dieser in sich geschlossenen Randlinie des Bewegungsbereichs kann dann ein Mittelpunkt zugeordnet werden. Alternativ kann ein Teil des Gelenks entlang zweier, insbesondere orthogonal zueinander liegender Hauptachsen bewegt werden und der Schnittpunkt dieser Hauptachsen wird als Mittelpunkt des Bewegungsbereichs definiert. Der vorgenannte Mittelpunkt des Bewegungsbereichs spannt nun zusammen mit dem Gelenkmittelpunkt (der insbesondere bei einer Relativbewegung der Gelenkteile ortsfest bleibt) eine Gerade auf, die die Orientierung des Gelenks darstellt.

Ist die Lage des Mittelpunkts des Drehgelenks nicht bekannt, so kann die Orientierung beispielsweise so festgelegt werden, dass der Rand des Bewegungsbereichs eine Ebene aufspannt und eine Mittelachse des Bewegungsbereichs normal zu dieser Ebene ist und durch den Mittelpunkt des durch den Rand umschriebenen Bereichs hindurch läuft. Auch können die Gelenke entlang jeweils einer Hauptbewegungsrichtung bewegt werden, wobei diese Richtungen beispielsweise senkrecht zueinander stehen können. Die durch die Bewegung aufgespannten Ebenen schneiden sich und die Schnittgerade kann als die Orientierung des Gelenks definiert werden.

Die Bewegbarkeit der ersten anatomischen Körperstruktur zur zweiten anatomischen Körperstruktur und somit die Bewegbarkeit des anatomischen Gelenks wird erfindungsgemäß insbesondere gemäß eine der folgenden Varianten beschrieben:
a) Durch den Mittelpunkt eines Bewegungsbereichs, der sich bei der Relativbewegung des ersten Teils des anatomischen Gelenks zu dem zweiten Teil des anatomischen Gelenks ergibt, wobei zur Definition der Orientierung insbesondere der Mittelpunkt mit einem anderen Punkt, der bei der Bewegung zur Bestimmung des Bewegungsbereichs seine Lage nicht ändert, insbesondere dem Drehpunkt, verbunden wird, so dass sich eine Linie ergibt, die die Orientierung des anatomischen Gelenks beschreibt.
b) Durch die Angabe absoluter Winkel der Flexion und/oder Extension und/oder Abduktion und/oder Adduktion. Dabei wird vorzugsweise die Mitte des Extensions-/Flexions-Winkelbereichs und/oder die Mitte des Abduktions-/Adduktions-Winkelbereichs verwendet, um so die Orientierung zu definieren. Alternativ oder zusätzlich kann auch die neutrale Relativlage des anatomischen Gelenks bestimmt werden (siehe unten).
c) Durch das Verhältnis von Winkeln, die die Gelenkbewegungs-Winkelbereiche relativ zu einer neutralen Relativlage beschreiben, in der die Körperstrukturen eine vorbestimmte Relativlage einnehmen, beispielsweise das Verhältnis des Flexionswinkels zu dem Extensionswinkel des anatomischen Gelenks und/oder das Verhältnis des Abduktionswinkels zu dem Adduktionswinkel. Die Orientierung des anatomischen Gelenks hängt von der neutralen Relativlage und den Winkelverhältnissen ab. Wird beispielsweise die Orientierung eines Implantatgelenks durch die Mittelachse des Bewegungsbereichs des Implantatgelenks beschrieben, so kann durch die vorgenannte Information bestimmt werden, wie die Mittelachse geändert werden muss, so dass sich bei neutraler Relativlage der Körperstrukturen die gewünschten Winkelverhältnisse einstellen, falls das künstliche Gelenk das anatomische Gelenk ersetzt.

Vorzugsweise werden die vorgenannten Winkel und/oder Winkelverhältnisse vor der Operation oder interoperativ durch eine Analyse des Bewegungsbereichs (ROM = range of motion) bestimmt. Alternativ oder ergänzend zur vorgenannten Bestimmung kann, insbesondere im Fall der Definition der Winkelverhältnisse die Definition der Orientierung auch auf einer statistischen Studie basieren. Beispielsweise kann aus der statistischen Studie ein bevorzugtes Winkelverhältnis für die Flexion zur Extension und/oder für die Abduktion zur Adduktion sich ergeben. So kann man, ohne eine Vorabbewegungsanalyse durchzuführen, die Orientierung des künstlichen Gelenks bestimmen.

Die Daten über die Bewegbarkeit des künstlichen Gelenks umfassen dann insbesondere Daten, die die Orientierung des künstlichen Gelenks betreffen, also wie beispielsweise Daten über den Bewegungsbereich, insbesondere über die Winkelbereiche der Flexion und/oder Extension und/oder Abduktion und/oder Adduktion und/oder besonders bevorzugt Daten über die Verhältnisse des Flexionswinkels zum Extensionswinkel und/oder des Abduktionswinkels zum Adduktionswinkel. Die Erfinder haben festgestellt, dass insbesondere die Verwendung der Verhältnisse als Grundlage zur Bestimmung einer geeigneten Orientierung eine besonders einfach zu handhabende und dennoch verlässliche Ausgangsbasis für die Bestimmung der Orientierung darstellt.

Durch die vorliegende Erfindung wird die relative Position des einen Gelenkteils zum anderen Gelenkteil berücksichtigt. Beispielsweise bei der Implantation eines Hüftgelenks kann so auch die relative Beziehung des Schafts zur Pfanne bei der Positionierung des Implantats berücksichtigt werden.

Wie oben ausgeführt, kann aus dem Bewegungsbereich und insbesondere den relativen Lagen zwischen der ersten und zweiten Körperstruktur ein Mittelpunkt des Gelenks bestimmt werden (der bei den Relativbewegungen ortsfest ist). Dieser Mittelpunkt kann zur Bestimmung einer geeigneten Lage zur Implantation des künstlichen Gelenks herangezogen werden. Es kann also bestimmt werden, welche Lage der Drehpunkt des künstlichen Gelenks einnehmen sollte. Insbesondere kann diese Lage so gewählt werden, dass sie mit dem Drehgelenkmittelpunkt des anatomischen Gelenks übereinstimmt. Die geeignete Lage kann also durch die Position des Drehgelenkmittelpunkts im Raum, also in einem der vorgenannten Bezugssysteme bestimmt werden.

Vorzugsweise wird die geeignete Orientierung unter bestimmten Randbedingungen bestimmt, wie z.B. dass der Bewegungsbereich des künstlichen Gelenks den Bewegungsbereich des anatomischen Gelenks umfasst oder zumindest in etwa entspricht. "Zumindest in etwa" bedeutet, dass falls der Bewegungsbereich durch Winkel beschrieben wird, eine Abweichung der beiden Bewegungsbereiche voneinander kleiner ab 20°, insbesondere kleiner ab 10°, 5°, oder 2° ist. Auf diese Art und Weise, ist gewährleistet, dass durch Bewegungen der ersten Körperstruktur relativ zur zweiten Körperstruktur kein Anschlag an die Grenzen des möglichen Bewegungsbereiches des künstlichen Gelenks erzielt werden kann. Ein derartiger Anschlag kann Komplikationen hervorrufen. Vorzugsweise erfolgt die Platzierung des künstlichen Gelenks dergestalt, dass die Orientierung des künstlichen Gelenks, also insbesondere die Mittelachse eines möglichen Bewegungsbereichs des künstlichen Gelenks (z.B. in einer bestimmten Ebene der Bewegung) mit der Orientierung, also insbesondere der Mittelachse des Bewegungsbereichs des anatomischen Gelenks (in einer entsprechenden Ebene) zumindest in etwa übereinstimmt. Dabei soll "zumindest in etwa" bedeuten, dass eine Abweichung der Mittelachsen voneinander kleiner als 20%, insbesondere kleiner als 10%, insbesondere kleiner als 5%, insbesondere kleiner als 2% des möglichen Bewegungsbereiches ist. Für das letzte genannte Beispiel von 2% würde dies bei einem maximalen Bewegungsbereich des künstlichen Gelenks von 100° bedeuten, dass die Abweichung der Mittelachse des Bewegungsbereichs des künstlichen Gelenks von der Mittelachse des Bewegungsbereichs des anatomischen Gelenks kleiner als +/- 2° ist. Die Mittelachse geht durch den Mittelpunkt des Gelenks sowie den Mittelpunkt des Bewegungsbereichs.

Alternativ oder zusätzlich zu der oben genannten Randbedingung der Bestimmung der geeigneten Orientierung anhand der Bewegungsbereiche des anatomischen und künstlichen Gelenks kann die Bestimmung der geeigneten Orientierung wie im Folgenden dargestellt erfolgen. Dabei wird basierend auf dem Implantat-Datensatz, die Informationen über den Widerstand oder des Risikos einer Luxation enthalten, die geeignete Lage bestimmt. Insbesondere sind Informationen über eine Trennung des ersten Teils von dem zweiten Teil insbesondere für bestimmte relative Lagen des ersten Teils relativ zum zweiten Teil enthalten. Vorzugsweise wird bei der Bestimmung der geeigneten Orientierung ein Wert für einen Widerstand gegen eine unerwünschte Trennung berücksichtigt. Dieser vorbestimmte Wert eines Widerstandes oder gewünschten Widerstandes kann sich insbesondere an dem typischen Widerstand eines anatomischen Gelenks gegen eine Luxation in einer vorbestimmten relativen Lage der ersten Körperstruktur relativ zur zweiten Körperstruktur orientieren. Insbesondere kann der Widerstandwert einer anatomischen Datenbank entnommen werden. Insbesondere kann er so gewählt werden, dass er mit einem typischen anatomischen Wert für einen Widerstand übereinstimmt oder zumindest in etwa übereinstimmt, wobei der Begriff "zumindest in etwa" und die damit verbundenen Abweichungen vom dem typischen Wert im oben definierten Sinne verstanden werden kann und beispielsweise kleiner als 20 % sind. Ein anatomischer Wert für einen Widerstand kann experimentell bestimmt werden und kann so aufgefasst werden, dass er einem Gegendruck entspricht, den eine intakte Kapsel und die Bänder des Acetabulum einer Luxation entgegensetzen.

Vorzugsweise werden die anatomischen Daten, insbesondere die Daten über die relativen Lagen der ersten und zweiten Körperstruktur und/oder mögliche Bewegungsbereiche mit anatomischen Daten aus einer Datenbank verglichen, die übliche (typische) oder maximal mögliche Bewegungsbereiche und/oder relative Lagen beschreiben, wie sie beim Menschen oder bei einem bestimmten Tier (üblicherweise) gegeben sind. Auf diese Art und Weise kann eine Plausibilitätsprüfung der eingegebenen anatomischen Daten durchgeführt werden. Insbesondere kann ein Warnsignal ausgegeben werden, falls die Überprüfung ergibt, dass die Daten von den üblichen Daten abweichen bzw. nicht innerhalb eines üblichen Wertebereichs liegen.

Vorzugsweise wird mindestens eine geeignete Orientierung bestimmt. Es kann also auch mehr als eine geeignete Orientierung bestimmt werden. Insbesondere umfasst der Begriff "mindestens eine Orientierung" auch, dass Bereiche für eine geeignete Orientierung bestimmt werden. Ein Beispiel für Bereiche sind beispielsweise Winkelbereiche (beispielsweise bezogen auf Symmetrieebenen), in denen eine Implantation eines künstlichen Gelenks als geeignet angesehen wird. Beispielsweise können mehrere Winkel oder ein Winkelbereich für die Implantation eines künstlichen Gelenks dergestalt geeignet sein, dass der maximal mögliche Bewegungs-Winkelbcreich des anatomischen Gelenks innerhalb des maximalen möglichen Bewegangs-Winkelbereichs des künstlichen Gelenks liegt. Derartige mehrere Orientierungen oder Orientierungsbereiche können insbesondere mittels einer Anzeigeeinrichtung (z.B. Monitor) oder allgemein einer Benutzerschnittstelle ausgegeben werden, so dass ein Bediener, insbesondere Operateur eine Orientierung aus den vorgeschlagenen Lagen auswählen kann oder eine Orientierung innerhalb des vorgeschlagenen Orientierungsbereichs auswählen kann. Die mehreren Orientierungen oder Orientierungsbereiche können auch basierend auf weiteren Nebenbedingungen, wie z.B. Minimierung des Risikos einer Luxation (siehe oben) eingeschränkt werden bzw. auf eine geeignete Lage reduziert werden.

Gemäß einer Ausführungsform der Erfindung können mehrere Implantat-Datensätze bereitgestellt werden, wobei jeder Implantat-Datensatz ein anderes künstlichen Gelenk betrifft. Die verschiedenen künstlichen Gelenke können insbesondere unterschiedliche Bewegbarkeiten aufweisen. Basierend auf den anatomischen Daten kann dann ein geeignetes künstliches Gelenk (mit dem diesen entsprechenden Implantat-Datensatz) oder mehrere geeignete künstliche Gelenke (mit den diesen entsprechenden Implantat-Datensätzen) ausgewählt werden.

Vorzugsweise wird ein künstliches Gelenk ausgewählt werden, für das der maximal mögliche Bewegungsbereich größer ist als der maximal mögliche Bewegungsbereich des zu ersetzenden anatomischen Gelenks. Die Auswahl kann automatisch erfolgen oder einem Benutzer können beispielsweise Vorschläge unterbreitet werden, so dass dieser dann die endgültige Auswahl trifft.

Das vorgehend beschriebene Verfahren zum Bestimmen einer Orientierung kann insbesondere bei einem erfindungsgemäßen Planungsverfahren zum Planen einer Implantation eines künstlichen Gelenks eingesetzt werden. Ein Bediener, beispielsweise ein Chirurg kann basierend auf dem Implantat-Datensatz und den Körperstruktur-Daten eine für eine Implantation geeignete Orientierung gemäß dem erfindungsgemäßen Verfahren bestimmen und somit basierend auf der geeigneten Orientierung die Implantation des künstlichen Gelenks planen. Die Bestimmung erfolgt dabei vorzugsweise so, dass nach Implantation des künstlichen Gelenks mit der geeigneten Orientierung das implantierte Gelenk alle relativen Lagen des ersten Teils relativ zum zweiten Teil einnehmen kann, die auch die erste und zweite Körperstruktur aufgrund des anatomischen Gelenks einnehmen konnten.

Die vorgenannten Verfahren können als ein Programm implementiert werden, das insbesondere auf einem computerlesbaren Speichermedium (z.B. CD oder DVD) gespeichert ist oder über eine Signalwelle (z.B. Internet-Downloadübertragung) übertragbar ist.

Weiter betrifft die vorliegende Erfindung eine Vorrichtung zum Bestimmen der Orientierung eines anatomischen Gelenks und/oder einer für eine Implantation geeigneten Orientierung des künstlichen Gelenks, die insbesondere einen Speicher insbesondere zum Speichern des mindestens einen Implantat-Datensatzes und zum Speichern der Körperstruktur-Daten umfasst. Weiter umfasst sie eine Datenverarbeitungseinrichtung, die ausgebildet ist, um Schritte des vorgenannten Verfahrens durchzuführen, insbesondere um eine Bestimmung, insbesondere eine Berechnung der Orientierung des anatomischen Gelenks und/oder der geeigneten Orientierung des künstlichen Gelenks durchzuführen, wobei hierbei insbesondere numerische Verfahren und/oder analytische Verfahren eingesetzt werden können, um mathematisch die Orientierung (des anatomischen und/oder künstlichen Gelenks) basierend auf den Körperstruktur-Daten und vorzugsweise auch auf dem mindestens einen Implantat-Datensatz zu bestimmen. Sind mehrere Implantat-Datensätze vorhanden, wird vorzugsweise zuerst eine Einschränkung der Implantat-Datensätze beispielsweise auf einen Implantat-Datensatz vorgenommen, wie dies bereits oben beschrieben wurde. In einem nächsten Berechnungsvorgang (oder nächsten Schritt) wird dann die Orientierung (des anatomischen und/oder künstlichen Gelenks) bestimmt, wobei der ausgewählte Implantat-Datensatz, der dem ausgewählten künstlichen Gelenk entspricht, verwendet wird.

Vorzugsweise umfasst die Vorrichtung auch eine Detektionseinrichtung, um die Körperstruktur-Daten zumindest teilweise zu bestimmen. Die Detektionseinrichtung ist dabei vorzugsweise so ausgebildet, dass sie Markereinrichtungen detektieren kann, die an die erste und/oder zweite Körperstruktur angebracht sind. Auf diese Art und Weise kann bei Bewegung der ersten Körperstruktur relativ zur zweiten Körperstruktur (bzw. umgekehrt) der Bewegungsbereich und/oder mögliche Lagen der ersten Körperstruktur relativ zur zweiten Körperstruktur bestimmt werden. Diese so erhaltenen Daten können dann als Körperstruktur-Daten bei der Bestimmung der Orientierung (des anatomischen und/oder künstlichen Gelenks) verwendet werden.

Vorzugsweise ist die Vorrichtung so ausgebildet, dass sie eine Benutzerschnittstelle, insbesondere eine Hinweiseinrichtung (z.B. Bildschirm) umfasst, die ausgebildet ist, dem Benutzer bei der Bewegung zumindest einer von den beiden Körperstrukturen (erste und zweite Körperstruktur) Hinweise zu geben. Insbesondere ist die Hinweiseinrichtung so ausgebildet, dass sie den Benutzer auf noch fehlende Bewegungen der Körperstrukturen hinweist und/oder dem Benutzer Hinweise gibt, in welche Richtungen die Bewegungen zu erfolgen haben. Ist beispielsweise bisher nur eine Bewegung in einer Extensions/Flexions-Richtung erfolgt, so kann die Hinweiseinrichtung (z.B. Bildschirm) den Bediener darauf hinweisen, dass eine Bewegung in einer Abduktions/Adduktions-Richtung noch fehlt, um ausreichend Körperstrukturdaten zur Bestimmung einer Orientierung zu haben. Insbesondere kann der Bediener aufgefordert werden, einzugeben, wenn eine relative Lage zwischen einer ersten Körperstruktur und einer zweiten Körperstruktur eine Extremlage oder Schlüssellage darstellt, die z.B. dem Rand eines möglichen Bewegungsbereiches der ersten Körperstruktur relativ zur zweiten Körperstruktur entspricht.

Bei der folgenden detaillierten Beschreibung werden weitere Merkmale der vorliegenden Erfindung offenbart. Merkmale verschiedener Ausführungsformen können miteinander kombiniert werden.
Figur 1 erläutert den Bewegungsbereich eines künstlichen Gelenks;
Figur 2 erläutert einen Luxationswiderstand;
Figur 3 zeigt schematisch den Aufbau einer erfindungsgemäßen Vorrichtung.

Bei der folgenden detaillierten Beschreibung wird beispielhaft als anatomisches Gelenk ein Hüftgelenk beschrieben. Insbesondere wird dabei der Bewegungsbereich des Hüftgelenks, genauer der gegebene Bewegungsbereich des (anatomischen) Hüftgelenks eines Patienten zur Bestimmung einer geeigneten Orientierung des künstlichen Gelenks verwendet.

Während eines chirurgischen Eingriffes für eine Totalendoprothese eines Hüftgelenks werden beide Teile der anatomischen Hüfte, die das anatomische Gelenk bewegen, durch zwei Teile eines künstlichen Gelenks (durch ein zweiteiliges Implantat) ersetzt. Das Acetabulum (Gelenkpfanne) innerhalb des Beckenknochens (Pelvis) wird durch ein Gelenkpfannenimplantat ersetzt. Der proximale Teil des Oberschenkelknochens wird durch eine im Folgenden Schaftimplantat genannte Implantatskombination aus Schaft und Gelenkkopf ersetzt. Der Schaft wird hierbei meist in den proximalen Oberschenkelknochen eingesetzt, während der Gelenkkopf meist auf den Schaft aufgesetzt wird.

Der Erfolg des chirurgischen Eingriffs hängt von der Stabilität des implantierten künstlichen Gelenks im Knochen (Becken bzw. Oberschenkelknochen) ab und hängt insbesondere auch von dem Bewegungsbereich ab, der von dem neuem künstlichen Gelenk ermöglicht wird. Der Bewegungsbereich kann die Stabilität beeinflussen. Ein zu geringer Bewegungsbereich verursacht das Anstoßen eines Teils des künstlichen Gelenks an dem anderen Teil des künstlichen Gelenks und kann folglich dazu führen, dass die Stabilität des Implantats, d.h. die Verbindung des Implantats mit den Knochen leidet. Dies kann dazu führen, dass sich das Implantat lockert oder vollständig vom Knochen löst. Die damit für den Patienten verbundenen Komplikationen sind insbesondere ein begrenzter Bewegungsbereich, Schmerzen, das Risiko einer Dislokation und schließlich das Risiko frühzeitig sich einer erneuten Operation unterziehen zu müssen.

Die vorliegende Erfindung hilft dem Chirurgen dabei, eine möglichst gute Orientierung des künstlichen Gelenks, insbesondere der künstlichen Gelenkpfanne und/oder des künstlichen Gelenkkopfes zu finden, um somit einen möglichst guten Bewegungsbereich zu erzielen. Vorzugsweise wird zum Bestimmen der möglichst optimalen Orientierung und insbesondere zum Platzieren des künstlichen Gelenks der Bewegungsbereich des anatomischen Gelenks zugrunde gelegt. Dieser Bewegungsbereich des anatomischen Gelenks kann als Datensatz bereitgestellt werden, kann vor dem chirurgischen Eingriff bestimmt werden oder kann während des chirurgischen Eingriffes bestimmt werden. Basierend auf den Daten über den Bewegungsbereich (ROM für "range of motion") wird dann die Lage der künstlichen Gelenkpfanne und/oder des künstlichen Schaftimplantats, also von zwei Teilen des Implantats bestimmt, insbesondere die Platzierung mit der geeigneten Orientierung geplant und erfolgt insbesondere die Platzierung während des chirurgischen Eingriffes mit der als geeignet bestimmten Orientierung.

Gemäß der Erfindung ist es nicht erforderlich, sich bei der Bestimmung der geeigneten Orientierung an Ebenen des Beckens zu orientieren, obwohl dies natürlich ebenfalls durchgeführt werden kann. Orientiert man sich an den Ebenen eines Beckens, so werden zwei Winkel definiert, die sich auf die Ebenen des Beckens beziehen, nämlich die Inklination und die Anteversion. In der Literatur wird eine so genannte sichere Zone zur Implantation einer künstlichen Gelenkpfanne definiert. Diese sichere Zone beträgt beispielsweise 40° bis 60° für die Inklination und 10° bis 30° für die Anteversion. Die Zonen sind so gewählt, dass die Wahrscheinlichkeit einer Fehlorientierung minimiert ist. Die angegebenen Werte sind jedoch nur statistische Werte und sind nicht an die gegebene Anatomie des individuellen Patienten angepasst. Dies kann dazu führen, dass eine Implantation, die sich an den Werten der sicheren Zone orientiert, für einen individuellen Patienten nicht optimal sein kann. Weiter ist es erforderlich, die Ebenen, insbesondere Symmetrieebenen des Beckens zu bestimmen. Dies kann schwierig sein, falls sich der Patient in lateraler Position befindet, da es erforderlich ist, im Anterior-Superior-Bereich der kontra lateralen Iliak-Spina einen Punkt (ASIS-Punkt) detektieren zu müssen. Berücksichtigt man erfindungsgemäß den individuellen Bewegungsbereich des anatomischen Gelenks (eines bestimmten Patienten), so können ungewünschte und insbesondere schmerzhafte Spannungen des Ligaments, insbesondere der Bänder vermieden oder vermindert werden. Insbesondere erlaubt die vorzugsweise Berücksichtigung des individuellen anatomischen Bewegungsbereichs des anatomischen Gelenks die Berücksichtigung von relativen Extremlagen der beiden Teile des anatomischen Gelenks, insbesondere die Berücksichtigung maximaler Bewegungsbereiche von Schlüsselbewegungen. Vorzugsweise wird weiter sowohl eine geeignete Orientierung zum Platzieren der künstlichen Gelenkpfanne als auch zum Platzieren des künstlichen Schaftimplantats bestimmt und insbesondere bei der Planung des chirurgischen Eingriffes berücksichtigt. Da die Orientierung des Schaftimplantats und der Gelenkpfanne miteinander in Beziehung stehen, kann durch die Bestimmung der Orientierung für beide Teile des künstlichen Gelenks eine suboptimale Platzierung eines Teils, insbesondere des Schaftimplantats verhindert werden, insbesondere kann ein beschränkter Bewegungsbereich vermieden werden. Gemäß einer Ausführungsform der Erfindung wird der Bewegungsbereich des Hüftgelenks intraoperativ bestimmt, um die gewonnenen Daten dann beim Planen einer Orientierung zum Implantieren der Gelenkpfanne und/oder des Schaftimplantats zu nutzen. Um die geeignete Orientierung zur Implantation des künstlichen Gelenks zu bestimmen, werden insbesondere die folgenden beiden Analysekonzepte eingesetzt, die auch miteinander kombiniert werden können.

Gemäß einem Konzept werden die maximalen Bewegungsbereiche von Schlüsselbewegungen bestimmt. Dabei kann es sich beispielsweise um Flexions-/Extensionsbewegungen, eine interne/externe Rotation oder eine Adduktions-/Abduktiotis-Bewegung oder andere kombinierte Bewegungen, wie z.B. eine interne Rotation während einer Flexionsbewegung handeln. Gemäß einem anderen Konzept stützt man sich auf eine für eine Dislokation (Luxation) kritische Richtung der Bewegung. Analysiert man den Bewegungsbereich eines anatomischen Gelenks, so werden vorzugsweise Daten gewonnen, die zumindest bei einem der vorgenannten Konzepte genutzt werden können, so dass die gewonnenen Daten verwendet werden können, um eine geeignete Lage für eine Gelenkpfanne und/oder ein Schaftimplantat zu bestimmen, um hierauf basierend eine Implantation des künstlichen Gelenks zu planen. Ein Operateur kann dann die Gelenkpfanne und/oder das Schaftimplantat basierend auf der Planung an der bestimmten geeigneten Position platzieren, indem insbesondere ein Navigationssystem verwendet wird, bei dem beispielsweise Markereinrichtungen an den Teilen des künstlichen Gelenks und/oder an den Körperstrukturen, in die die Teile des künstlichen Gelenks zu implantieren sind, angebracht sind. Die Markereinrichtungen werden vorzugsweise von dem Navigationssystem detektiert, um die Lage der Teile des künstlichen Gelenks zu bestimmen, insbesondere relativ zu den vorgenannten Körperstrukturen zu bestimmen, um so den Operateur während der Operation wertvolle Informationen über die Lage, insbesondere relative Lage der beteiligten Körperstrukturen und des künstlichen Gelenks zu liefern.

Ein Registrierungsverfahren, das z.B. mithilfe einer Detektionseinrichtung, die Markereinrichtungen detektiert und insbesondere mithilfe eines Navigationssystems durchgeführt werden kann, umfasst z.B. mindestens einen der folgenden Registrierungsschritte:
a) Eine Markereinrichtung (z.B. Referenzstern) wird zum Erfassen der Lage und insbesondere zum Verfolgen der Bewegung der Lage des Beckens und/oder des Oberschenkelknochens an dem Becken und/oder dem Oberschenkelknochen angebracht.
b) Die Lage eines ASIS-Punktes wird z.B. mithilfe eines Pointers erfasst. Alternativ oder zusätzlich kann z.B. mittels eines Pointers ein Punkt auf dem Schambein erfasst werden. Ein Pointer ist ein Zeigegerät, an dem mindestens zwei Markerkugeln angebracht sind und für den die relative Lage zwischen der Spitze des Pointers und der Markerkugeln bekannt ist, so dass mit der Spitze Landmarken und insbesondere Punkte abgegriffen und deren Lage durch Detektion der Markerkugeln mittels einer Detektionseinrichtung, insbesondere durch ein Navigationssystem erfasst und somit registriert werden können.
c) Das Bein des Patienten wird in eine neutrale Lage gebracht. Diese Position des Oberschenkelknochens gegenüber dem Becken wird als neutrale Lage abgespeichert.
d) Landmarken eines Oberschenkels und/oder den Oberschenkel kennzeichnende geometrische Eigenschaften, wie z.B. Symmetrieebenen oder Symmetrieachsen, beispielsweise die Achse des Schaftes des Oberschenkelknochens oder die Achse des Oberschenkelhalses werden beispielsweise mit Markereinrichtungen, Pointern und/oder durch Diagnoseeinrichtungen (wie z.B. CT oder Röntgenaufnahmen) bestimmt.
e) Der Bewegungsbereich des Oberschenkelknochens, insbesondere relativ zu dem Becken und/oder in einem vorbestimmten Bezugssystem (für das bereits oben Beispiele gegeben wurden) wird bestimmt. Die Bestimmung erfolgt insbesondere für eine vorbestimmte anatomische Situationen, wie z.B. die maximale Flexion, die maximale Extension, die maximale Abduktion, die maximale Adduktion und für kombinierte Bewegungen.
f) Oberflächenpunkte des Acetabulum (Hüftgelenkpfanne) werden erfasst.

Das unter b) Aufgeführte wird insbesondere verwendet, um ein skaliertes Beckenmodell bereitzustellen. Das oben unter c) Aufgeführte wird insbesondere eingesetzt, um eine Ausgangssituation oder Null-Situation zu definieren. Diese Ausgangssituation kann dann verwendet werden, um als Bezugspunkt für Schlüsselbewegungen (wie z.B. Flexionsbewegung, Extensionsbewegung, Adduktionsbewegung oder Abduktionsbewegung) zu dienen. Insbesondere kann die Ausgangssituation als Bezugspunkt für die Ränder des maximalen Bewegungsbereichs dienen. Die Ausgangssituation wird ebenfalls vorzugsweise gespeichert und dient insbesondere zur Bestimmung der Beinlänge und zur Berechnung eines Versatzes (Offset).

Der obige Punkt e) kann durch folgendes optionales Merkmal noch ergänzt werden. Die Erfassung des Bewegungsbereichs kann durch Plausibilitätsüberprüfungen abgesichert werden. Ein Beispiel hierfür stellt ein kleinster akzeptabler bzw. üblicher Flexionsbereich dar, wie er beim Menschen üblicherweise gegeben ist. Z.B. kann der kleinste noch akzeptable Flexionsbereich 110° betragen. Ein Bediener kann durch Warnhinweise darauf hingewiesen werden, falls die vorgenannten Plausilibitätskriterien nicht erfüllt sind, also falls beispielsweise ein Flexionsbereich kleiner als 110° festgestellt wird.

Das erfindungsgemäße Verfahren und die erfindungsgemäße Vorrichtung, insbesondere ein Programm, das, wenn es auf einem Computer ausgeführt wird oder in einem Computer geladen wird, das erfindungsgemäße Verfahren durchführt, kann so gestaltet sein, dass es den Bediener unterstützt, wenn dieser die anatomischen Daten erfasst oder ergänzt. Insbesondere kann der Bediener (z.B. Chirurg) dabei von dem Programm unterstützt werden, wenn er noch nicht den Bewegungsbereich des anatomischen Gelenks voll bzw. ausreichend erfasst hat. Insbesondere kann basierend auf statistischen Modellen das Programm den Bediener unterstützen, unvollständige Körperstrukturdaten zu vervollständigen, so dass insbesondere die möglichen Bewegungen, bevorzugt Bereiche von Schlüsselbewegungen erfasst sind.

Vorzugsweise wird demnach basierend auf zumindest einem der folgenden beiden Analysekonzepte eine geeignete Orientierung für das künstliche Gelenk oder einem Teil davon, z.B. für die Gelenkpfanne berechnet:
a) Maximaler Bereich von Schlüsselbewegungen.
b) Kritische Richtung für eine Dislokation (Luxation)

Insbesondere sind die beiden obigen Konzepte auf eine Platzierung mit geeigneter Orientierung der Gelenkpfanne in einem ersten Schritt gerichtet. Da jedoch die Implantation der Gelenkpfanne und die Implantation des Schaftimplantats miteinander verbunden sind, umfasst vorzugsweise eine vollständige Planung ebenso die Bestimmung einer geeigneten Orientierung des Schaftimplantats. Während die Orientierung der Gelenkpfanne z.B. relativ zur Mediansagittalebene der Patienten angegeben werden kann, wird die Orientierung des Schaftimplantats (insbesondere des Schaftteils des Schaftimplantats) beispielsweise relativ zu einer Längsachse des Oberschenkelknochens angegeben. Wenn einmal eine geeignete Orientierung für die Gelenkpfanne bestimmt worden ist, kann basierend hierauf eine geeignete, dazu passende Orientierung des Schaftimplantats bestimmt werden, um den gewünschten Bewegungsbereich des Gelenks nach der Implantation zu gewährleisten. Vorzugsweise wird das Verfahren so gestaltet, dass nach Implantation eines Teils des künstlichen Gelenks, also beispielsweise der Gelenkpfanne, die Orientierung des implantierten Teils überprüft wird und insbesondere gemessen wird. Basierend auf der gemessenen Orientierung kann dann eine neue Berechnung oder eine Aktualisierung der bisher vorgenommenen Berechnung durchgeführt werden, falls die gemessene Orientierung nicht mit der vorher erfindungsgemäß bestimmten Orientierung übereinstimmt, also etwas davon abweicht. Somit kann die Bestimmung der geeigneten Orientierung für das zweite implantierte Teil des künstlichen Gelenks an die tatsächliche Orientierung des zuerst implantierten Teils des künstlichen Gelenks angepasst werden. Somit kann beispielsweise erreicht werden, dass trotz der Abweichung der Bewegungsbereich des künstlichen Gelenks den Bewegungsbereich des anatomischen Gelenks umfasst. Insbesondere kann auch die Orientierung des Schaftimplantats zuerst bestimmt werden und dann in einem zweiten Schritt die Orientierung der Gelenkpfanne. Insbesondere kann die Orientierung des Schaftimplantats nach Implantation im Oberschenkel verifiziert werden und die verifizierte (d.h. gemessene) Orientierung kann dann als Grundlage zur Berechnung der geeigneten Lage für die Gelenkpfanne genommen werden. Auch kann das Schaftimplantat zuerst implantiert werden und seine Orientierung verifiziert werden und danach eine Implantation der Gelenkpfanne erfolgen.

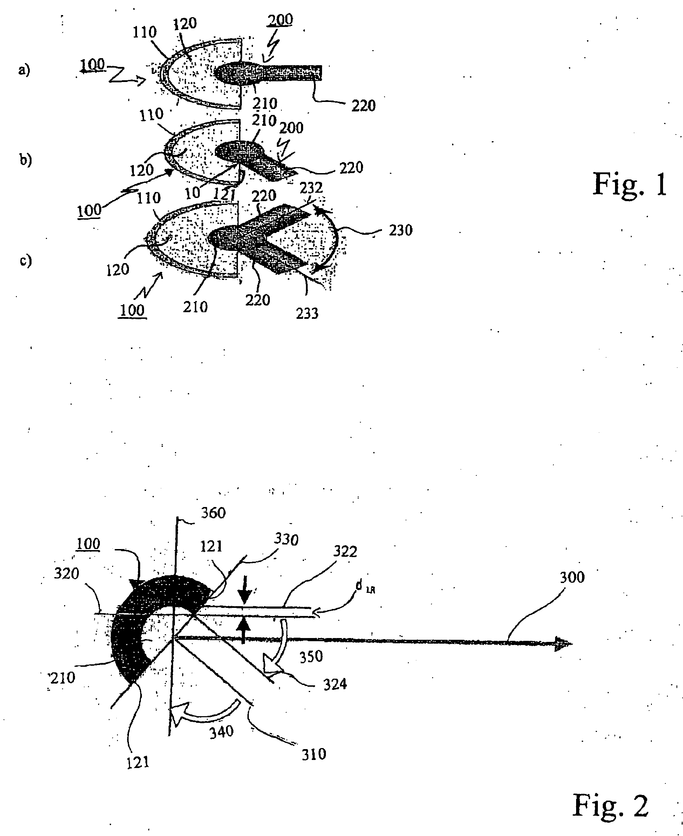

Hinsichtlich des oben als a) bezeichneten Analysekonzepts wird noch Folgendes vorgebracht. Vorteilhafterweise berechnet eine Software die Maximalbereiche von Schlüsselbewegungen, z.B. die Bereiche von Flexions-/Extensionsbewegungen und Abduktions-/Adduktionsbewegungen. Diese Bereiche werden dann verwendet, um die bestmögliche Kombination einer Gelenkpfanne mit einem Gelenkkopf auszuwählen bzw. festzulegen. Verfügbare Kombinationen werden vorteilhafterweise von einer Datenbank bereitgestellt. Figur 1 zeigt ein Beispiel einer Kombination aus einem ersten und zweiten Teil eines künstlichen Gelenks 100, 200, die ein Maximalbereich einer Bewegung entlang eines Primärbogens 230 (so genannter "primary arc range") von 100° bereitstellt. Vorzugsweise werden die Gelenkpfannen 100 dergestalt implantiert, also in eine derartige Lage gebracht, dass der Maximalbereich ohne ein Anstoßen 10 erreicht werden kann. Ein "Anstoßen" 10 bedeutet hier, dass ein Teil 200 des künstlichen Gelenks im Rahmen einer Gelenkbewegung so anstößt, dass eine weitere Fortführung der Gelenkbewegung verhindert wird. Vorzugsweise wird also eine Ausgangslage oder Null-Lage des künstlichen Gelenks 100, 200 so platziert, dass diese in der Mitte des Maximalbereichs oder der Maximalbereiche des anatomischen Gelenks liegt. Die Figur 1a zeigt das künstliche Gelenk 100, 200 in einer derartigen Ausgangsposition. Die Figur 1b zeigt eine Situation, bei der der Rand eines Bewegungsbereichs erreicht ist, also ein Teil 200 des künstlichen Gelenks im Rahmen einer Gelenkbewegung an dem anderen Teil 100 (121) anstößt bzw. anliegt. Genauer stößt der Übergangsbereich zwischen Gelenkkugel 210 und Gelenkschaft 220 an der Endfläche 121 der Gelenkpfanne 100 bei dem Punkt 10 an. Die Gelenkpfanne 100 besteht aus einer äußeren Schale 110, in die ein zur Gelenkkugel passendes Futter 120 *(auch Liner genannt)* eingepasst ist. Figur 1c zeigt einen durch die Anstoßpunkte 10 definierten maximalen Bewegungsbereich 230 (primary arc range), der beispielsweise 100° betragen kann. Im Folgenden wird ein Beispiel vorgestellt:

Die maximale Flexion im Bereich zur Ausgangsposition beträgt beispielsweise 80°. Die maximale Extension verglichen zu der Ausgangssituation beträgt 15°. Dies führt zu einem maximalen Flexions-/Extensionsbereich von 95°. Da dieser Bereich von 95° kleiner ist als der maximale Bewegungsbereich von 100° (primary arc range 100°) des künstlichen Gelenks der Figur 1, kann dieses künstliche Gelenk verwendet werden. Die Mitte dieses maximalen Flexions-/Extensionsbereichs beträgt demnach 47,5°. Diese Mitte beschreibt in einer Ebene die geeignete Orientierung für eine Platzierung der Gelenkpfanne hinsichtlich der Flexions-/Extensionsebene. Berechnet man diese geeignete Orientierung in Bezug auf die Ausgangssituation (Null-Situation), so würde dies einer Orientierung entsprechen, die zumindest 32,5° Flexion entspricht. Dies errechnet sich auch aus dem Mittelwert des maximalen Flexions-/Extensionsbereich von 47,5° minus dem maximalen Extensionsbereich von 15°.

Hinsichtlich des oben genannten Analysekriteriums b) wird noch Folgendes vorgebracht. Es wird davon ausgegangen, dass Daten gegeben sind, die die maximale Abduktion beschreiben. Beispielsweise ein Programm oder das erfindungsgemäße Verfahren verwendet diese Daten und berechnet darauf basierend die Richtung einer für diesen Fall möglichen Luxation. Dabei wird für diesen Fall angenommen, dass die Luxation entlang der Oberschenkelhalsachse des Femurs erfolgt. Die Oberschenkelhalsachse kann insbesondere aus gegebenen anatomischen Daten bestimmt werden, wie sie beispielsweise durch ein Computertomogramm (CT) ermittelt werden können. Somit ist die Lagebeziehung zwischen der maximalen Abduktionslage und der Oberschenkelhalsachse bekannt. Weiter kann vorzugsweise angenommen werden, dass für den Fall der Luxation kein Anstoßen zwischen dem ersten und zweiten Teil des künstlichen Gelenks, also in diesem Beispiel zwischen Kopf und Pfanne gegeben ist. Weiter sind vorzugsweise die geometrischen Daten, also insbesondere Daten über Form und/oder Gestalt des künstlichen Gelenks aus einer Datenbank bekannt. Weiter kann man annehmen, dass ein Teil des künstlichen Gelenks, z.B. eine Mittelachse der Gelenkpfanne und die Richtung der Luxation einen Winkel einschließen, der im Folgenden Neigungswinkel genannt wird. Ein mechanischer Widerstand gegen die Luxation hängt von einem Abstand ab, der im Folgenden Luxationskenngröße d_{LR} genannt wird. Dieser Widerstand hängt von dem Neigungswinkel ab. Dies ist näher in Figur 2 erläutert. In Figur 2 ist die Gelenkpfanne 100 gezeigt, in der eine halbkugelförmige Öffnung für den Kopf 210 eines Gelenkkopfes ist. Dieser Gelenkkopf 210 kann sich im Zustand der Verbindung mit der Gelenkpfanne 100 von der Gelenkpfanne 100 lösen. Dies wird als Luxation bezeichnet. Die Richtung der Luxation ist in Figur 2 mit 300 bezeichnet. Für diese Luxationsrichtung können verschiedene Richtungen angenommen werden. Vorzugsweise werden Richtungen angenommen, wie sie sich für Schlüssellagen des einen Teils des Gelenks relativ zu dem anderen Teil ergeben. Beispielsweise kann die Luxationsrichtung für den Zustand der maximalen Abduktion bestimmt werden. Insbesondere kann die Luxationsrichtung für diese Lage oder für andere Schlüssellagen bzw. Lagen so berechnet werden, dass angenommen wird, dass eine Luxation entlang der Oberschenkelhalsachse erfolgt. Somit kann die Luxationsrichtung identisch mit der Oberschenkelhalsachse für den Fall der maximalen Abduktion sein.

Vorzugsweise sind die geometrischen Daten für das künstliche Gelenk, insbesondere für ein ausgewähltes künstliches Gelenk aus einer Vielzahl von künstlichen Gelenken bekannt, also insbesondere in einer Datenbank gespeichert. Die Symmetrieachse, um die die Gelenkpfanne 100 der Figur 2 rotationssymmetrisch ist, ist in Figur 2 mit dem Bezugszeichen 310 bezeichnet. Diese Mittelachse 310 geht durch den Mittelpunkt der schalenförmigen Vertiefung der Gelenkpfanne 100 und steht insbesondere senkrecht auf einer Ebene 330, die die Endflächen 121 der Gelenkpfanne 100 verbindet. Die Mittelachse 310 entspricht der Orientierung des künstlichen Gelenks.

Eine so genannte Luxationsachse 320 ist parallel zur Richtung der Luxation 300. Sie ist weiterhin senkrecht zu einer Geraden 360, die senkrecht zur Richtung der Luxation ist und durch die Mediansagittalebene (vom Kopf zum Fuß) verläuft. Die angenommene Luxationsrichtung ist senkrecht dazu. Sie entspricht im Zustand der maximalen Abduktion der Oberschenkelhalsachse. Die Gerade 360 schneidet einen Schnittpunkt zwischen der Mittelachse 310 und der Ebene 330. Die Luxationsachse 320 schneidet weiterhin die Ebene 330 an einem Randpunkt der Endfläche 121 der Gelenkpfanne 100, und zwar den Randpunkt der Endfläche 121, der der Mittelachse 310 am nächsten ist. Der Winkel zwischen der Mittelachse 310 und der Geraden 360, die senkrecht zu der Luxationsachse 320 ist, ist der Neigungswinkel der Gelenkpfanne 100 und ist in Figur 2 mit 340 bezeichnet. Der Luxations-Neigungswinkel 350 ist ebenfalls in Figur 2 bezeichnet und ist der Winkel zwischen der Luxationsachse 320 und einer Geraden 324, die senkrecht zur Ebene 330 ist und die Ebene 330 an dem oben genannten Randpunkt der Endfläche 121 schneidet.

Der Überstand zwischen der Implantatkante und dem höchsten Punkt der Implantataushöhlung (in dem entsprechenden Fall) wird im Folgenden als Luxationskenngröße d_{LR} bezeichnet und führt zum mechanischen Widerstand, der der Luxation entgegengesetzt wird. Bei diesem Beispiel wird die Luxationskenngröße durch den Abstand d_{LR} angegeben, wobei der sich aus dem Abstand ergebende Widerstand um so größer ist je größer dieser Abstand ist. Der Abstand d_{LR} ist der Abstand zwischen der Luxationsachse 320 und einer dazu parallelen Geraden 322. Diese parallele Gerade 322 schneidet die Gerade 360, die senkrecht zur Luxationsachse 320 ist und die die Ebene 330 in der Mitte zwischen den Endflächen 121 schneidet.

In der Bestimmung des Wertes der benötigten Luxationskenngröße können neben geometrischen Eigenschaften des künstlichen Gelenks auch beispielsweise Materialeigenschaften des künstlichen Gelenks mit einbezogen werden, insbesondere Oberflächenreibungseigenschaften und Elastizität des Materials. Je elastischer bzw. flexibler das Material des künstlichen Gelenks ist, um so leichter kann eine Luxation bewirkt werden. Je geringer die Reibung zwischen dem einen Teil des künstlichen Gelenks und dem anderen Teil des künstlichen Gelenks, also beispielsweise zwischen Gelenkkopf und Gelenkpfanne, ist, umso leichter kann der eine Teil von dem anderen Teil getrennt werden.

Die Luxationskenngröße d_{LR} und, wie oben dargelegt, folglich der Neigungswinkel der Luxation wird erfindungsgemäß insbesondere dergestalt optimiert, dass eine maximale Abduktion ermöglicht wird, wobei es kein Anstoßen des einen Teil des Gelenks an den anderen gibt, und wobei weiter ein möglichst hoher Widerstand gegen eine Luxation gewährleistet ist.

Wenn einmal der optimale Widerstand, insbesondere der sich aus den geometrischen Eigenschaften ergebende Widerstand, gefunden wurde, so ist somit ein Luxations-Neigungswinkel 350 festgelegt, und aus diesem kann ein geeigneter Neigungswinkel 340 der künstlichen Gelenkpfanne und somit eine diesem Neigungswinkel entsprechende, geeignete Orientierung des künstlichen Gelenks bestimmt werden. In maximaler Abduktion wird die Luxationsrichtung festgelegt. Die Ausrichtung der Körperstrukturen Pelvis und Femur sind in der Position bekannt. Aus z.B. der Datenbank weiß man, welche minimale Luxationskenngröße d_{LR} man haben muss, um nicht zu luxieren. Diese minimalste Luxationskenngröße d_{LR} ergibt einen Neigungswinkel zwischen der Mittelachse 310 des künstlichen Gelenks und der Luxationsachse. Beispielsweise entspricht die Luxationsachse der Oberschenkelhalsachse oder die Beziehung zwischen der Luxationsachse und der Oberschenkelhalsachse ist bekannt (z.B. weil der Gelenkkopf bereits implantiert ist). Über die Oberschenkelhalsachse und den Neigungswinkel kann nun eine geeignete Lage der Mittelachse 310 und somit eine geeignete Orientierung des Gelenkpfannenimplantats ermittelt werden.

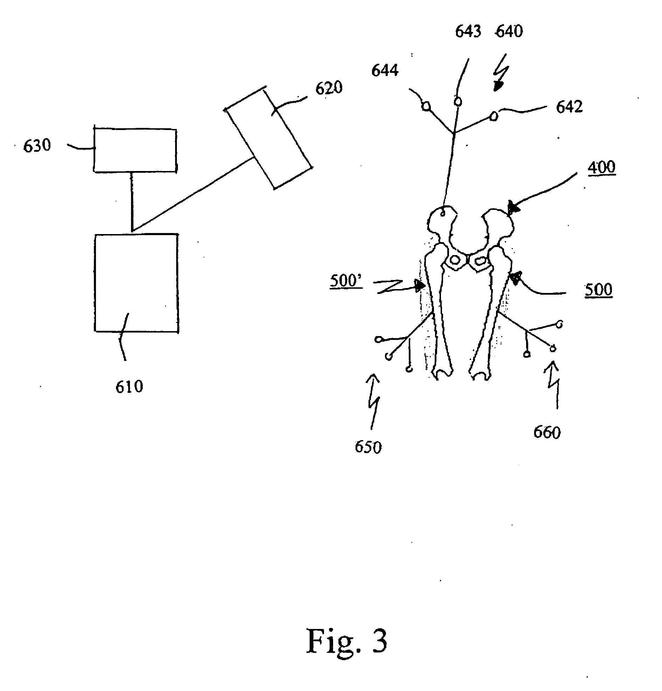

Figur 3 zeigt eine erfindungsgemäße Vorrichtung einer Datenverarbeitungseinrichtung 610. Sie ist mit einem Monitor 630 und einer Detektionseinrichtung 620 verbunden. Die Detektionseinrichtung 620 ist ausgebildet, Markereinrichtungen 640, 650 und 660 zu detektieren, die wie abgebildet jeweils drei Kugeln aufweisen, die mit 642, 643 und 644 bezeichnet sind.

Die Markereinrichtungen sind am Becken und an jeweils einem Oberschenkelknochen angebracht. Wird der Oberschenkelknochen 500 bzw. 500' relativ zum Becken 400 bewegt, so können somit mögliche relative Lagen der Oberschenkelknochen 500 und 500' relativ zum Becken 400 erfasst werden. Insbesondere können typische Schlüssellagen und Extremlagen (Maximallagen) am Patienten, insbesondere während der Operation, erfasst werden, bevor eine Implantation durchgeführt wird.

Basierend auf den erfassten Daten kann dann ein künstliches Gelenk, beispielsweise eine Gelenkpfanne und eine Gelenkkopf implantiert werden. Insbesondere kann das künstliche Gelenk, insbesondere jeder Teil des künstlichen Gelenks ebenfalls mit einer Markereinrichtung versehen sein, um die exakte und gewünschte Platzierung zu gewährleisten. Die Vorrichtung kann ausgebildet sein, Hinweise für den Operateur zu geben, in welche Lage der Oberschenkelknochen 500 bzw. 500' relativ zu dem Becken 400 noch zu bringen ist, um ausreichend Datenmaterial zur Berechnung einer geeigneten Lage des künstlichen Gelenks zu haben. Insbesondere kann die Einrichtung 610, 620 und 630 auch dazu genutzt werden, das künstliche Gelenk nach Bestimmung der geeigneten Orientierung und insbesondere entsprechend dem Ergebnis des erfindungsgemäßen Planungsverfahrens zu platzieren, indem der Operateur durch Hinweise geleitet wird und/oder indem ein Implantationsroboter gesteuert wird.

Wie oben ausgeführt, liegt eine besonders bevorzugte Ausgestaltung der vorliegenden Erfindung darin, dass Verhältnisse der Flexion zur Extension und der Abduktion zur Adduktion gebildet werden, um so eine geeignete Orientierung des künstlichen Gelenks zu bestimmen, wobei die geeignete Orientierung des künstlichen Gelenks insbesondere auch über ein derartiges Verhältnis festgelegt wird.

Man kann beispielsweise annehmen, dass durch Messung oder durch eine statistische Analyse einer Vielzahl anatomischer Gelenke sich folgende die Bewegbarkeit des anatomischen Gelenks beschreibende Körperstruktur-Daten ergeben haben:
Flexion = 120°
Extension = 10°
Abduktion = 40°
Adduktion = 20°.

Diese absoluten Winkelwerte werden relativ zur neutralen Relativlage des Beins zum Becken bestimmt. Die neutrale Relativlage wird mit Hilfe von Referenzsternen am Femur und am Becken erkannt, wobei sie so definiert wird, dass in der neutralen Relativlage der Femur nicht rotiert ist (keine Innenrotation und keine Außenrotation) und dass das Bein entlang einer Achse, vorzugsweise der mechanischen Achse des Beins ausgerichtet wird. Die mechanische Achse ist die Achse, in der die Kraft bei einem stehenden Menschen wirkt. Die neutrale Relativlage kann somit auf einfache Weise von der Operation erfasst werden. Die mechanische Achse des Beins ist in der Medizin beispielsweise so definiert, dass sie im zweibeinigen Stand vom Zentrum des Hüftknochens, durch das Zentrum des Knies zum Mittelpunkt des Fußgelenks verläuft und insbesondere einen Winkel von drei Grad gegenüber der Vertikalen aufweist. Die neutrale Relativlage kann auch durch das Abgreifen von Landmarken bestimmt werden. Zur Bestimmung der neutralen Relativlage kann einfach, auch ohne Messung von Landmarken das Bein entlang der mechanischen Achse ausgerichtet werden. Die relative Lage zwischen Femur und Becken wird detektiert und gespeichert. Vorzugsweise wird die neutrale Relativlage durch die in Figur 3 gezeigte Vorrichtung gemessen. Sie wird vorzugsweise gespeichert. Die neutrale Relativlage ist vorzugsweise ebenfalls Bestandteil der Köperstruktur-Daten. Dies insbesondere dann, wenn nur Winkelverhältnisse aber nicht absolute Winkelwerte Bestandteil der Körperstruktur-Daten sind.

Die obigen absoluten Winkelwerte erfüllen die folgenden Winkelverhältnisse:
Flexion/Extension = 120°/10° = 12/1
Abduktion/Adduktion = 40°/20° = 2/1.

Gemäß der besonders bevorzugten Ausführungsform genügen nun die obigen Winkelverhältnisse (im Beispiel 12/1 und 2/1) dazu, die Position (Orientierung) des Gelenks genau und eindeutig festzulegen, wenn man von einer neutralen Relativlage des Beins ausgeht. Die neutrale Relativlage ist vorzugsweise eine leicht bestimmbare Relativlage, z.B. das stehende Bein, wie oben ausgeführt wurde.

Beispielsweise kann man zur Planung (virtuell) oder Operation (real) folgende Schritte durchführen:
a) Das Bein wird virtuell oder real in die neutrale Relativlage gebracht. Virtuell kann dies erfolgen, da zuvor die Lagedaten des Beins für die neutrale Relativlage gespeichert wurden, wie oben beschrieben wurde. Diese Lagedaten für die neutrale Relativlage werden insbesondere zu Beginn der Operation insbesondere mittels Referenzsterne erfasst, indem beispielsweise das Bein entlang der mechanischen Achse ausgerichtet wird. Die Lagedaten werden einem Datenverarbeitungssystem zur Planung bereitgestellt.
b) Real oder virtuell wird die relative Lage des Femurs relativ zum Becken, also die neutrale Relativlage beibehalten. Dies bedeutet, dass die Daten, die die neutrale Relativlage beschreiben, konstant gehalten werden. Geändert wird aber die relative Lage zwischen den Gelenkteilen, also beispielsweise wird bei ortsfestem Femur und insbesondere bei ortsfestem Schaftimplantat oder ortsfester Oberschenkelhalsachse das Pfannenimplantat (real oder virtuell) relativ zum Becken bewegt, um die relative Lage der Schenkelhalsachse oder der Halsachse des Oberschaftimplantats relativ zur Achse des Pfannenimplantats zu ändern, bis sich die gewünschten Winkelverhältnisse für die neutrale Relativlage ergeben.

Die neutrale Achse des Pfannenimplantats entspricht der Mittelachse 310 und somit der Orientierung des Pfannenimplantats (also des Gelenks). Die Ebene, mit der sich das Acetabulumgelenk öffnet, also die Ebene 330 hat theoretisch einen Öffnungswinkel von 180° und wird durch die neutrale Achse gleichmäßig in 90° und 90° aufgeteilt. Es ergibt sich also ein Verhältnis 1/1 für die neutrale Achse des Pfannenimplantats. Beispielsweise aber nicht obligatorisch kann die Referenzachse (also z.B. die Oberschenkelhalsachse oder die Halsachse des Schaftimplantats) geplant und insbesondere verifiziert werden So kann anfänglich und rein optional die Mittel-/Neutralachse des Acetabulumimplantats mit der Referenzachse ausgerichtet werden. Somit ergibt sich dann "Range of Motion"-Verhältnisse von 1/1. Ausgehend hiervon kann man dann das Pfannenimplantat bewegen, bis das gewünschte Winkelverhältnis in der neutralen Relativlage erreicht ist.

Die Einstellung der Winkelverhältnisse kann durch die folgenden beiden Schritte realisiert werden:
a) Um die gewünschten Verhältnisse zu erzielen, wird ein Gelenkteil (beispielsweise das Acetabulumimplantat) bewegt oder gedreht, während z.B. das andere Gelenkteil (z.B. Femurimplantat) ortsfest bleibt. Durch Bewegung oder Drehung des Pfannenimplantats wird eine Änderung in Richtung Flexion/Extension erreicht. Die Bewegung oder Drehung erfolgt so, dass das gewünschte Verhältnis (z.B. 12/1 für Flexion/Extension) erreicht wird (12/1 = 166,15°/13,85°). Die offene Ebene des Gelenkteils (Acetabulumimplantats) wird also nach der Bewegung oder Drehung durch die Referenzachse im gewünschten Verhältnis aufgeteilt. Die offene Ebene des Gelenkteils (Acetabulumimplants) wird also nach der Bewegung oder Drehung durch die Oberschenkelhalsachse oder die Halsachse des Implantats im gewünschten Verhältnis aufgeteilt, falls das Bein in der neutralen Relativlage ist. Denn die neutrale Achse des Gelenkteils (Mittelachse) dreht sich mit, wenn das Gelenkteil gedreht wird. Die Referenzachse für die Aufteilung kann die Oberschenkelhalsachse oder die Halsachse des Implantates sein, die beispielsweise durch die Planung bzw. die Verifizierung bestimmt wurde.
b) Vorzugsweise erfolgt eine Aufteilung der offenen Ebene des Gelenks für mindestens zwei festgelegte Winkelverhältnisse, also beispielsweise neben der Flexion/Extension auch noch zusätzlich für die AbduktionlAdduktion. Hierzu wird ein weiterer Schritt durchgeführt. Im genannten Beispiel würde dann im zweiten Schritt für die Abduktion/Adduktion das Verhältnis x/y z.B. 2/1 erzielt werden. Die offene Ebene des Acetabulumimplantats wäre also somit im Verhältnis x/y z.B. 2/1 durch die Referenzachse in der Abduktions-/Adduktions-Richtung unterteilt, also z.B. in 120°/60° unterteilt. Dadurch fließen in die Optimierung der Position des Pfannenimplantates mehrere Verhältnisse ein.

Die vorgenannten Schritte können im Rahmen eines Planungsverfahrens durchgeführt werden. Vorzugsweise sind Markereinrichtungen an der ersten und zweiten Körperstruktur (beispielsweise Femur und Acetabulum) angebracht. So kann mit Hilfe der erfindungsgemäßen Planung das gewünschte Winkelverhältnis oder können die gewünschten Winkelverhältnisse vom Operateur überwacht und mittels der erfindungsgemäßen Vorrichtung eingestellt werden. Alternativ kann auch ohne Erfassung der Lage der Referenzachse mit Pointern oder Markern die geeignete Orientierung überprüft werden, indem die erste und zweite Körperstruktur relativ zueinander bewegt wird und überprüft wird, ob sich nach der Implantation die gewünschten Verhältnisse ergeben. Falls nicht, kann eine Korrektur vorgenommen werden.

Insbesondere werden Instrumente mit Marker benutzt, um die Implantate zu plazieren. Dies ermöglicht es dann, die Implantate so einzusetzen wie es in der Planung vorgesehen war.

Desweiteren kann nach dem Einsetzen der Implantate eine Range of Motion-Analyse vorgenommen werden, bei der die zwei Körperstrukturen relativ zueinander bewegt werden. Diese liefert dann die Bewegungen (z.B. Flexion, Extension, ...), und man kann das Ergebnis der Operation und die Planung vergleichen.

## Patentansprüche

1. Verfahren zum Bestimmen einer Orientierung eines künstlichen Gelenks, insbesondere für die Planung einer Implantation von mindestens einem künstlichen Gelenk (100, 200) in einem menschlichen oder tierischen Körper, wobei ein erster Teil (100) des künstlichen Gelenks und ein zweiter Teil (200) des künstlichen Gelenks ausgebildet sind, miteinander eine künstliche Gelenkverbindung eingehen zu können, wobei der erste Teil (100) zur Implantation in einer ersten Körperstruktur (400) des menschlichen oder tierischen Körpers vorgesehen ist und der zweite Teil (200) zur Implantation in einer zweiten Körperstruktur (500, 500') des menschlichen oder tierischen Körpers vorgesehen ist, um ein anatomisches Gelenk (400, 500; 400, 500'), das die erste Körperstruktur (400) mit der zweiten Körperstruktur (500) verbindet oder verbunden hat, zu ersetzen, **dadurch kennzeichnend, dass** das Verfahren die folgenden Schritte umfasst:
- patientenspezifische Körperstruktur-Daten werden bereitgestellt, die eine Bewegbarkeit der ersten Körperstruktur (400) relativ zur zweiten Körperstruktur (500) und somit die Bewegbarkeit des anatomischen Gelenks beschreiben,
- basierend auf den Körperstruktur-Daten wird eine für die Implantation des künstlichen Gelenks geeignete Orientierung bestimmt.

2. Verfahren nach Anspruch 1, wobei die Bewegbarkeit durch mindestens ein erstes Winkelverhältnis beschrieben wird, das sich aus dem Verhältnis eines ersten Winkels zu einem zweiten Winkel ergibt, wobei der erste und zweite Winkel durch eine erste Bewegung eines ersten Teils des zu ersetzenden anatomischen Gelenks relativ zu einem zweiten Teil des zu ersetzenden anatomischen Gelenks bestimmbar sind, wobei die erste Bewegung in einer ersten Richtung und/oder in einer ersten Ebene von einem ersten Bewegungsendpunkt zu einem zweiten Bewegungsendpunkt erfolgt und der erste Winkel der Winkel zwischen dem ersten Bewegungsendpunkt und einer vordefinierten, zwischen den beiden Bewegungsendpunkten liegenden neutralen Relativlage ist und der zweite Winkel der Winkel zwischen dem zweiten Bewegungsendpunkt und der neutralen Relativlage ist.

3. Verfahren nach einem der Ansprüche 1 bis 2, bei welchem die Bewegbarkeit zusätzlich zumindest durch ein zweites Winkelverhältnis beschrieben wird, das sich aus dem Verhältnis eines dritten Winkels zu einem vierten Winkel ergibt und mit einer zweiten Bewegung bestimmbar ist, die in einer zweiten Richtung und/oder zweiten Ebene verläuft, die zumindest schräg und vorzugsweise zumindest in etwa orthogonal zur ersten Richtung und/oder Ebene ist, wobei die zweite Bewegung von einem dritten Bewegungsendpunkt zu einem vierten Bewegungsendpunkt erfolgt und der dritte Winkel der Winkel zwischen dem dritten Bewegungsendpunkt und der vordefinierten, zwischen den beiden Bewegungsendpunkten liegenden neutralen Relativlage ist und der vierte Winkel der Winkel zwischen dem vierten Bewegungsendpunkt und der neutralen Relativlage ist.

4. Verfahren nach Anspruch 2 oder 3, bei welchem die erste und/oder zweite Bewegung über den gesamten anatomisch möglichen Bewegungsbereich erfolgt.

5. Verfahren nach einem der Ansprüche 2 bis 4, bei welchem die neutrale Relativlage durch die Detektion von Landmarken der ersten und zweiten Körperstruktur bestimmt wird, wobei vorgegeben ist, dass die Landmarken in der neutralen Relativlage eine vorbestimmte Relativlage einnehmen.

6. Verfahren nach einem der Ansprüche 1 bis 5, bei welchem die Bewegbarkeit basierend auf einem Verhältnis des Flexionswinkels zum Extensionswinkel und/oder basierend auf einem Verhältnis des Abduktionswinkels zum Adduktionswinkel bestimmt wird.

7. Verfahren nach einem der vorhergehenden Ansprüche, das weiter den Schritt umfasst:
- mindestens ein Implantat-Datensatz wird bereitgestellt, der eine Bewegbarkeit des ersten Teil relativ zum zweiten Teils betrifft,
- wobei basierend auf den Körperstruktur-Daten und dem mindestens einen Implantat-Datensatz die geeignete Orientierung des künstlichen Gelenks bestimmt wird.

8. Verfahren nach dem vorhergehenden Anspruch, bei welchem der Implantat-Datensatz Folgendes umfasst:
- mindestens einen möglichen Bewegungsbereich (230) des mindestens einen künstlichen Gelenks (100, 200); und/oder
- mögliche Lagen (350) des ersten Teils relativ zum zweiten Teil; und/oder
- Daten über die Geometrie des künstlichen Gelenks; und/oder
- Daten (d_{LR}), die die Hemmung oder einen Widerstand gegen eine relative Bewegung des ersten und zweiten Teils betreffen.

9. Verfahren nach einem der vorhergehenden Ansprüche, bei welchem die durch die Körperstruktur-Daten umfasste Bewegbarkeit Folgendes umfasst:
- mindestens einen mit dem zu ersetzenden anatomischen Gelenk (400, 500, 500') oder mit einem gleichartigen Gelenk möglichen Bewegungsbereich; und/oder
- mögliche Lagen der ersten Körperstruktur (400) relativ zur zweiten Körperstruktur (500, 500'); und/oder
- Daten über die Geometrie des anatomischen Gelenks; und/oder
- Daten über die Hemmung einer oder über einen Widerstand gegen eine relative Bewegung der ersten und zweiten Körperstruktur; und/oder
- Daten über mindestens eine Luxationsrichtung.

10. Verfahren nach dem vorhergehenden Anspruch, bei welchem die Bestimmung der geeigneten Lage so ausgestaltet ist, dass im Falle der Platzierung des künstlichen Gelenks (100, 200) mit der geeigneten Orientierung der mögliche Bewegungsbereich des anatomischen Gelenks (400, 500, 500') durch den möglichen Bewegungsbereich des künstlichen Gelenks (100, 200) umfasst ist oder zumindest in etwa entspricht.

11. Verfahren nach einem der vorhergehenden Ansprüche, bei welchem die Bestimmung der geeigneten Orientierung so ausgestaltet ist, dass ein Widerstand gegen eine Luxation des künstlichen Gelenks optimiert wird und/oder der Widerstand dazu benutzt wird, die geeignete Orientierung zu bestimmen oder ein bereits als geeignet bestimmte Orientierung zu bewerten und/oder zu verbessern.

12. Verfahren nach einem der vorhergehenden Ansprüche, bei welchem die anatomischen Daten überprüft werden, indem sie mit vorgegebenen Daten verglichen werden, die anatomische übliche oder maximal mögliche Bewegbarkeiten und/oder mögliche relative Lagen der Körperstrukturen bei einem Menschen oder einem Tier darstellen.

13. Verfahren nach einem der vorhergehenden Ansprüche, bei welchem mehr als ein Implantat-Datensatz bereitgestellt wird, wobei jeder Implantat-Datensatz eines von verschiedenen künstlichen Gelenken (100, 200) beschreibt, und basierend auf den Körperstruktur-Daten und den Implantat-Datensätzen sowohl mindestens ein geeignetes künstliches Gelenk (100, 200) ausgewählt wird als auch die mindestens eine geeignete Orientierung für die Implantation des mindestens einen ausgewählten künstlichen Gelenks (100, 200) bestimmt wird.

14. Verfahren nach einem der vorhergehenden Ansprüche, bei welchem mittels eines Navigationssystems und/oder mittels einer Detektionseinrichtung (620) und/oder mittels medizinischer Analyse und/oder Diagnoseeinrichtungen die Körperstruktur-Daten zumindest teilweise bestimmt werden.

15. Verfahren nach einem der vorhergehenden Ansprüche, bei welchem zumindest eine Markereinrichtung (640) an der ersten und/oder zweiten Körperstruktur (400, 500, 500') angebracht wird und/oder angebracht ist und die Lage und/oder eine Bewegung der ersten und/oder zweiten Körperstruktur (400, 500, 500') durch Detektion der mindestens einen Markereinrichtung (640) erfasst wird, um die Körperstruktur-Daten zu bestimmen.

16. Planungsverfahren zum Planen einer Implantation eines zweiteiligen künstlichen Gelenks (100, 200) in einem menschlichen Körper, wobei das Planungsverfahren das Verfahren nach einem der vorhergehenden Ansprüche verwendet, um eine geeignete Orientierung für das Implantieren des künstlichen Gelenks (100, 200) zu bestimmen.

17. Computerprogramm, das, wenn es in einen Computer geladen wird, oder auf einem Computer läuft, den Computer veranlasst, die Schritte des Verfahrens oder Planungsverfahrens nach einem der vorhergehenden Ansprüche auszuführen.

18. Computerlesbares Speichermedium, auf dem das Computerprogramm nach dem vorhergehenden Anspruch gespeichert ist oder physikalische Signalwelle, die als Information das Programm nach dem vorhergehenden Anspruch trägt.

19. Vorrichtung zum Bestimmen einer für eine Implantation geeigneten Orientierung von mindestens einem künstlichen Gelenk (100, 200) in einem menschlichen oder tierischen Körper, wobei ein erster Teil (100) des künstlichen Gelenks und ein zweiter Teil (200) des künstlichen Gelenks ausgebildet sind, miteinander eine künstliche Gelenkverbindung eingehen zu können, wobei der erste Teil zur Implantation in einer ersten Körperstruktur (400) des menschlichen oder tierischen Körpers vorgesehen ist und der zweite Teil (500, 500') zur Implantation in einer zweiten Körperstruktur des menschlichen oder tierischen Körpers vorgesehen ist, um ein anatomisches Gelenk (400, 500; 400, 500'), das die erste Körperstruktur (400) mit der zweiten Körperstruktur (500) verbindet oder verbunden hat, zu ersetzen, **dadurch kennzeichnend, dass** die Vorrichtung Folgendes umfasst:
einen Speicher zum Speichern von patientenspezifischen Körperstruktur-Daten, die eine Bewegbarkeit der ersten (400) relativ zur zweiten Körperstruktur (500, 500') betreffen und somit die Beweglichkeit des anatomischen Gelenks; und
eine Datenverarbeitungseinrichtung (610), die ausgebildet ist, basierend auf den Körperstruktur-Daten eine für die Implantation des künstlichen Gelenks geeignete Orientierung zu bestimmen.

20. Vorrichtung nach dem vorhergehenden Anspruch, bei welcher weiter eine Detektionseinrichtung (620) vorgesehen ist, um die Körperstruktur-Daten zumindest teilweise zu bestimmen, wobei die Detektionseinrichtung (620) ausgebildet ist, die Lage und/oder eine Bewegung der ersten und/oder zweiten Körperstruktur (400, 500, 500') durch Detektion von mindestens einer Markereinrichtung (640), die an der ersten und/oder zweiten Körperstruktur (400, 500, 500') angebracht ist, zu erfassen.

21. Vorrichtung nach dem vorhergehenden Anspruch, die weiter eine Hinweiseinrichtung (630) umfasst, die ausgebildet ist, basierend auf den gespeicherten Lagen und/oder Bewegungen einen Bediener auf die Lagen und/oder Bewegungen der ersten und/oder zweiten Körperstruktur hinzuweisen, die noch zu detektieren sind, um zur Bestimmung der Orientierung des anatomischen Gelenks und/oder der geeigneten Orientierung des künstlichen Gelenks noch erforderliche Körperstruktur-Daten zu gewinnen.

## Claims

1. A method for determining an orientation of an artificial joint, in particular for planning an implantation of at least one artificial joint (100, 200) in a human or animal body, wherein a first part (100) of the artificial joint and a second part (200) of the artificial joint are designed to be able to form an artificial joint connection with each other, wherein the first part (100) is provided for implantation in a first body structure (400) of the human or animal body, and the second part (200) is provided for implantation in a second body structure (500, 500') of the human or animal body, in order to replace an anatomical joint (400, 500; 400, 500') which connects or connected the first body structure (400) to the second body structure (500), **characterising in that** the method comprises the following steps:
- patient-specific body structure data are provided which describe a mobility of the first body structure (400) relative to the second body structure (500) and thus the mobility of the anatomical joint;
- an orientation which is suitable for implanting the artificial joint is determined on the basis of the body structure data.

2. The method according to claim 1, wherein the mobility is described by at least a first angular ratio which follows from the ratio of a first angle to a second angle, wherein the first angle and the second angle can be determined by a first movement of a first part of the anatomical joint to be replaced relative to a second part of the anatomical joint to be replaced, wherein the first movement is performed in a first direction and/or in a first plane from a first movement end point to a second movement end point, and the first angle is the angle between the first movement end point and a predefined neutral relative location between the two movement end points, and the second angle is the angle between the second movement end point and the neutral relative location.

3. The method according to any one of claims 1 to 2, wherein the mobility is additionally described by at least a second angular ratio which follows from the ratio of a third angle to a fourth angle and can be determined by a second movement in a second direction and/or second plane which is at least oblique with respect to, and preferably at least approximately orthogonal to, the first direction and/or plane, wherein the second movement is performed from a third movement end point to a fourth movement end point, and the third angle is the angle between the third movement end point and the predefined neutral relative location between the two movement end points, and the fourth angle is the angle between the fourth movement end point and the neutral relative location.

4. The method according to claim 2 or 3, wherein the first and/or second movement is performed over the entire anatomically possible range of motion.

5. The method according to any one of claims 2 to 4, wherein the neutral relative location is determined by detecting landmarks on the first and second body structure, wherein it is predetermined that the landmarks assume a predetermined relative location in the neutral relative location.

6. The method according to any one of claims 1 to 5, wherein the mobility is determined on the basis of a ratio of the flexion angle to the extension angle and/or on the basis of a ratio of the abduction angle to the adduction angle.

7. The method according to any one of the preceding claims, further comprising the step of:
- providing at least one implant dataset relating to a mobility of the first part relative to the second part;
- wherein the suitable orientation of the artificial joint is determined on the basis of the body structure data and the at least one implant dataset.

8. The method according to the preceding claim, wherein the implant dataset comprises:
- at least one possible range of motion (230) of the at least one artificial joint (100, 200); and/or
- possible locations (350) of the first part relative to the second part; and/or
- data concerning the geometry of the artificial joint; and/or
- data (d_{LR}) relating to the inhibition of or a resistance against a relative movement of the first and second part.

9. The method according to any one of the preceding claims, wherein the mobility included in the body structure data comprises:
- at least one range of motion which is possible using the anatomical joint (400, 500, 500') to be replaced or using an identical joint; and/or
- possible locations of the first body structure (400) relative to the second body structure (500, 500'); and/or
- data concerning the geometry of the anatomical joint; and/or
- data concerning the inhibition of or a resistance against a relative movement of the first and second body structure; and/or
- data concerning at least one luxation direction.

10. The method according to the preceding claim, wherein determining the suitable location is embodied such that when the artificial joint (100, 200) is positioned with the suitable orientation, the possible range of motion of the anatomical joint (400, 500, 500') is included in the possible range of motion of the artificial joint (100, 200) or at least approximately corresponds to it.

11. The method according to any one of the preceding claims, wherein determining the suitable location is embodied such that a resistance against a luxation of the artificial joint is optimised and/or the resistance is used to determine the suitable orientation or to assess and/or improve an orientation which has already been determined to be suitable.

12. The method according to any one of the preceding claims, wherein the anatomical data are verified by comparing them with predetermined data which represent normal anatomical or maximum possible mobilities and/or possible relative locations of the body structures in a human being or an animal.

13. The method according to any one of the preceding claims, wherein more than one implant dataset is provided, wherein each implant dataset describes one of various artificial joints (100, 200), and on the basis of the body structure data and the implant datasets, at least one suitable artificial joint (100, 200) is selected and the at least one suitable orientation for implanting the at least one selected artificial joint (100, 200) is determined.

14. The method according to any one of the preceding claims, wherein the body structure data are at least partially determined by means of a navigation system and/or by means of a detection means (620) and/or by means of medical analysis and/or diagnostic means.

15. The method according to any one of the preceding claims, wherein at least one marker means (640) is attached to the first and/or second body structure (400, 500, 500'), and the location and/or a movement of the first and/or second body structure (400, 500, 500') is detected by detecting the at least one marker means (640), in order to determine the body structure data.

16. A planning method for planning an implantation of a two-part artificial joint (100, 200) in a human body, wherein the planning method uses the method according to any one of the preceding claims in order to determine a suitable orientation for implanting the artificial joint (100, 200).

17. A computer program which, when it is loaded onto a computer or is running on a computer, causes the computer to perform the steps of the method or planning method according to any one of the preceding claims.

18. A computer-readable storage medium on which the computer program according to the preceding claim is stored, or physical signal waves which carry the program according to the preceding claim as information.

19. A device for determining an orientation which is suitable for an implantation of at least one artificial joint (100, 200) in a human or animal body, wherein a first part (100) of the artificial joint and a second part (200) of the artificial joint are designed to be able to form an artificial joint connection with each other, wherein the first part (100) is provided for implantation in a first body structure (400) of the human or animal body, and the second part (200) is provided for implantation in a second body structure (500, 500') of the human or animal body, in order to replace an anatomical joint (400, 500; 400, 500') which connects or connected the first body structure (400) to the second body structure (500), **characterised in that** the device comprises:
- a memory for storing patient-specific body structure data which relate to a mobility of the first body structure (400) relative to the second body structure (500, 500') and thus describe the mobility of the anatomical joint; and
- a data processing means (610) which is designed to determine an orientation which is suitable for implanting the artificial joint, on the basis of body structure data.

20. The device according to the preceding claim, wherein a detection means (620) is also provided in order to at least partially determine the body structure data, wherein the detection means (620) is designed to detect the location and/or a movement of the first and/or second body structure (400, 500, 500') by detecting at least one marker means (640) attached to the first and/or second body structure (400, 500, 500').

21. The device according to the preceding claim, further comprising an indicating means (630) which is designed, on the basis of the stored locations and/or movements, to indicate to an operator the locations and/or movements of the first and/or second body structure which still have to be detected in order to obtain the body structure data still necessary in order to determine the orientation of the anatomical joint and/or the suitable orientation of the artificial joint.

## Revendications

1. Procédé pour déterminer une orientation d'une articulation artificielle, en particulier pour programmer une implantation d'au moins une articulation artificielle (100, 200) dans un corps humain ou animal, dans lequel une première partie (100) de l'articulation artificielle et une seconde partie (200) de l'articulation artificielle sont conçues de manière à pouvoir former ensemble une liaison artificielle d'articulation, dans lequel la première partie (100) est prévue pour être implantée dans une première structure corporelle (400) du corps humain ou animal, et la seconde partie (200) est prévue pour être implantée dans une seconde structure corporelle (500, 500') du corps humain ou animal, afin de remplacer une articulation anatomique (400, 500 ; 400, 500') qui relie ou a relié la première structure corporelle (400) à la seconde structure corporelle (500), **caractérisé en ce que** le procédé comporte les étapes suivantes consistant à :
- préparer des données de structure corporelle spécifiques à un patient, qui décrivent une mobilité de la première structure corporelle (400) par rapport à la seconde structure corporelle (500) et ainsi la mobilité de l'articulation anatomique,
- déterminer, sur la base des données de structure corporelle, une orientation qui convient à l'implantation de l'articulation artificielle.

2. Procédé selon la revendication 1, dans lequel on décrit la mobilité par au moins un premier rapport angulaire qui résulte du rapport d'un premier angle à un deuxième angle, dans lequel on peut déterminer les premier et deuxième angles par un premier mouvement d'une première partie de l'articulation anatomique à remplacer par rapport à une seconde partie de l'articulation anatomique à remplacer, dans lequel le premier mouvement est effectué dans une première direction et/ou dans un premier plan d'un premier point de fin de mouvement à un deuxième point de fin de mouvement, et le premier angle est l'angle entre le premier point de fin de mouvement et une position relative neutre prédéfinie entre les deux points de fin de mouvement, et le deuxième angle est l'angle entre le deuxième point de fin de mouvement et la position relative neutre.

3. Procédé selon l'une quelconque des revendications 1 à 2, dans lequel on décrit également la mobilité par au moins un second rapport angulaire qui résulte du rapport d'un troisième angle à un quatrième angle et qu'on peut déterminer par un second mouvement qui est effectué dans une seconde direction et/ou un second plan qui est au moins oblique par rapport, et de préférence au moins approximativement orthogonal, à la première direction et/ou au premier plan, dans lequel le second mouvement est effectué d'un troisième point de fin de mouvement à un quatrième point de fin de mouvement, et le troisième angle est l'angle entre le troisième point de fin de mouvement et la position neutre prédéfinie entre les deux points de fin de mouvement, et le quatrième angle est l'angle entre le quatrième point de fin de mouvement et la position relative neutre.

4. Procédé selon la revendication 2 ou 3, dans lequel le premier et/ou second mouvement est effectué sur toute la plage de mouvement anatomiquement possible.

5. Procédé selon l'une des revendications 2 à 4, dans lequel on détermine la position relative neutre par la détection de points de repère de la première et de la seconde structure corporelle, dans lequel on prédétermine que les points de repère occupent une position relative prédéfinie dans la position relative neutre.

6. Procédé selon l'une quelconque des revendications 1 à 5, dans lequel on détermine la mobilité sur la base d'un rapport de l'angle de flexion à l'angle d'extension et/ou sur la base d'un rapport de l'angle d'abduction à l'angle d'adduction.

7. Procédé selon l'une quelconque des revendications précédentes, comportant en outre l'étape consistant à :
- préparer au moins un ensemble de données d'implant concernant une mobilité de la première partie par rapport à la seconde partie,
- dans lequel on détermine, sur la base des données de structure corporelle et du au moins un ensemble de données d'implant, l'orientation qui convient à l'articulation artificielle.

8. Procédé selon la revendication précédente, dans lequel l'ensemble de données d'implant comporte ce qui suit :
- au moins une plage de mouvement possible (230) de la au moins une articulation artificielle (100, 200), et/ou
- des positions possibles (350) de la première partie par rapport à la seconde partie, et/ou
- des données relatives à la géométrie de l'articulation artificielle, et/ou
- des données (d_{LR}) concernant le blocage ou une résistance à l'égard d'un mouvement relatif de la première et de la seconde partie.

9. Procédé selon l'une quelconque des revendications précédentes, dans lequel la mobilité incluse dans les données de structure corporelle comporte ce qui suit :
- au moins une plage de mouvement possible avec l'articulation anatomique à remplacer (400, 500, 500') ou avec une articulation similaire, et/ou
- des positions possibles de la première structure corporelle (400) par rapport à la seconde structure corporelle (500, 500'), et/ou
- des données relatives à la géométrie de l'articulation anatomique, et/ou
- des données relatives au blocage ou à une résistance à l'égard d'un mouvement relatif de la première et de la seconde structure corporelle, et/ou
- des données relatives à au moins une direction de luxation.

10. Procédé selon la revendication précédente, dans lequel la détermination de la position qui convient est conçue de telle sorte que, lorsque l'articulation artificielle (100, 200) est placée avec l'orientation qui convient, la plage de mouvement possible de l'articulation anatomique (400, 500, 500') est incluse dans la plage de mouvement possible de l'articulation artificielle (100, 200) ou correspond au moins approximativement à celle-ci.

11. Procédé selon l'une quelconque des revendications précédentes, dans lequel la détermination de l'orientation qui convient est conçue de telle sorte qu'une résistance à l'égard d'une luxation de l'articulation artificielle est optimisée et/ou la résistance est utilisée pour déterminer l'orientation qui convient, ou pour évaluer et/ou améliorer une orientation déjà déterminée comme convenable.

12. Procédé selon l'une quelconque des revendications précédentes, dans lequel on vérifie les données anatomiques en les comparant à des données prédéfinies qui représentent des mobilités anatomiques usuelles ou maximales possibles et/ou des positions relatives possibles des structures corporelles chez un homme ou un animal.

13. Procédé selon l'une quelconque des revendications précédentes, dans lequel on prépare plus d'un ensemble de données d'implant, chaque ensemble de données d'implant décrivant l'une des différentes articulations artificielles (100, 200), et, sur la base des données de structure corporelle et des ensembles de données d'implant, on sélectionne au moins une articulation artificielle (100, 200) qui convient et on détermine également la au moins une orientation qui convient pour l'implantation de la au moins une articulation artificielle (100, 200) sélectionnée.

14. Procédé selon l'une quelconque des revendications précédentes, dans lequel on détermine au moins en partie les données de structure corporelle, au moyen d'un système de navigation et/ou au moyen d'un dispositif de détection (620) et/ou au moyen d'une analyse médicale et/ou de dispositifs de diagnostic.

15. Procédé selon l'une quelconque des revendications précédentes, dans lequel on place au moins un dispositif de repérage (640) sur la première et/ou la seconde structure corporelle (400, 500, 500'), et/ou celui-ci est placé, et on relève la position et/ou un mouvement de la première et/ou de la seconde structure corporelle (400, 500, 500') par détection du au moins un dispositif de repérage (640) afin de déterminer les données de structure corporelle.

16. Procédé de programmation pour programmer une implantation d'une articulation artificielle (100, 200) en deux parties dans un corps humain, le procédé de programmation utilisant le procédé selon l'une quelconque des revendications précédentes pour déterminer une orientation convenable pour l'implantation de l'articulation artificielle (100, 200).

17. Programme d'ordinateur qui, lorsqu'il est chargé dans un ordinateur ou tourne sur un ordinateur, fait exécuter à l'ordinateur les étapes du procédé ou du procédé de programmation selon l'une quelconque des revendications précédentes.

18. Support de mémoire lisible par un ordinateur sur lequel est mémorisé le programme d'ordinateur selon la revendication précédente, ou onde de signaux physiques qui porte le programme selon la revendication précédente comme information.

19. Dispositif pour déterminer une orientation convenant à une implantation d'au moins une articulation artificielle (100, 200) dans un corps humain ou animal, dans lequel une première partie (100) de l'articulation artificielle et une seconde partie (200) de l'articulation artificielle sont conçues de manière à pouvoir former ensemble une liaison artificielle d'articulation, dans lequel la première partie est prévue pour être implantée dans une première structure corporelle (400) du corps humain ou animal, et la seconde partie (500, 500') est prévue pour être implantée dans une seconde structure corporelle du corps humain ou animal, afin de remplacer une articulation anatomique (400, 500 ; 400, 500') qui relie ou a relié la première structure corporelle (400) à la seconde structure corporelle (500), **caractérisé en ce que** le dispositif comporte ce qui suit :
une mémoire pour mémoriser des données de structure corporelle spécifiques à un patient, qui concernent une mobilité de la première structure corporelle (400) par rapport à la seconde structure corporelle (500, 500') et donc la mobilité de l'articulation anatomique, et
un dispositif de traitement de données (610) qui est conçu pour déterminer, sur la base des données de structure corporelle, une orientation qui convient pour l'implantation de l'articulation artificielle.

20. Dispositif selon la revendication précédente, dans lequel on prévoit en outre un dispositif de détection (620) pour déterminer au moins en partie les données de structure corporelle, dans lequel le dispositif de détection (620) est conçu pour relever la position et/ou un mouvement de la première et/ou de la seconde structure corporelle (400, 500, 500') par détection d'au moins un dispositif de repérage (640) qui est placé sur la première et/ou la seconde structure corporelle (400, 500, 500').

21. Dispositif selon la revendication précédente, comportant en outre un dispositif de signalisation (630) qui est conçu pour signaler à un opérateur, sur la base des positions et/ou mouvements mémorisés, les positions et/ou mouvements de la première et/ou seconde structure corporelle qui doivent encore être détectés, afin d'obtenir encore des données de structure corporelle nécessaires pour déterminer l'orientation de l'articulation anatomique et/ou de l'orientation convenable de l'articulation artificielle.
